# FASCICULE DE BREVET EUROPEEN

(11) **EP 3 619 533 B1**
(45) Date de publication et mention de la délivrance du brevet: **28.09.2022**
(21) Numéro de dépôt: 18721048.9
(22) Date de dépôt: 04.05.2018
(51) Int. Cl.: G01N 33/50, G01N 33/569

(54) **PROCÉDÉ DE DÉTECTION D'UNE RÉPONSE CELLULAIRE IMMUNE**
ERKENNUNGSVERFAHREN EINER ZELLULÄREN IMMUNANTWORT
METHOD FOR DETECTING A CELLULAR IMMUNE RESPONSE

(30) Priorité: 05.05.2017 EP 17169653; 21.09.2017 EP 17192308
(43) Date de publication de la demande: 11.03.2020
(73) Titulaire: bioMérieux, 69280 Marcy l'Etoile (FR)
(72) Inventeur: ATAMAN-ONAL, Yasemin, 01600 Reyrieux (FR); ARNAUD, Michel, 69100 Villeurbanne (FR); BERTHIER, Franck, 69830 Saint Georges de Reneins (FR); DUPRET-CARRUEL, Jacqueline, 69006 Lyon (FR); PASSAGOT, Jacques, 69210 Saint Pierre la Palud (FR)
(86) Numéro de dépôt international: PCT/EP2018/061533
(87) Numéro de publication internationale: WO 2018/202864

(56) Documents cités:
- EP-B1- 1 144 447
- US-A1- 2015 153 361
- NEUBAUER J C ET AL: "Towards standardized automated immunomonitoring: an automated ELISpot assay for safe and parallelized functionality analysis of immune cells", CYTOTECHNOLOGY, KLUWER ACADEMIC PUBLISHERS, DORDRECHT, NL, vol. 69, no. 1, 28 novembre 2016 (2016-11-28), pages 57-73, XP036144122, ISSN: 0920-9069, DOI: 10.1007/S10616-016-0037-4 [extrait le 2016-11-28]
- ALMEIDA C A M ET AL: "Automation of the ELISpot assay for high-throughput detection of antigen-specific T-cell responses", JOURNAL OF IMMUNOLOGICAL METHODS, ELSEVIER SCIENCE PUBLISHERS B.V.,AMSTERDAM, NL, vol. 344, no. 1, 15 mai 2009 (2009-05-15), pages 1-5, XP027566131, ISSN: 0022-1759 [extrait le 2009-03-10]
- SYLVIA JANETZKI ET AL: "Guidelines for the automated evaluation of Elispot assays", NATURE PROTOCOLS, vol. 10, no. 7, 25 juin 2015 (2015-06-25), pages 1098-1115, XP055385167, GB ISSN: 1754-2189, DOI: 10.1038/nprot.2015.068
- S JANETZKI: "Automation of the elispot technique: Past, present, and future", JOURNAL OF THE ASSOCIATION FOR LABORATORY AUTOMATION, vol. 9, no. 1, 1 février 2004 (2004-02-01) , pages 10-15, XP055385615, ISSN: 1535-5535, DOI: 10.1016/S1535-5535(03)00084-4

## Description

La présente invention concerne le domaine de la détection des réponses cellulaires immunes. En particulier, l'invention concerne un nouveau procédé de détection d'une réponse cellulaire immune dans un échantillon biologique, ladite réponse étant utile tant dans le domaine du diagnostic que dans le domaine de la thérapie, notamment pour les études de toxicité de futurs médicaments et vaccins.

Le domaine de la détection des réponses cellulaires immunes est un domaine en plein essor depuis ces dernières années. Ainsi, avec la mise au point du test ELIspot (Enzyme-linked immunospot) dans les années 1980, utilisé à l'origine pour mesurer la sécrétion des immunoglobulines par les cellules B d'un organisme humain, puis élargi à toutes cellules sanguines circulantes capables de sécréter une molécule après leur stimulation *in vitro,* même présentes en quantité faible, un champ d'application médicale large s'est ouvert, notamment pour mieux comprendre et suivre la réponse immunitaire, par exemple dans le cadre des maladies infectieuses ou des maladies auto-immunes, et dans le domaine du cancer (Cox J.H. et al., 2006).

La réponse immunitaire, provoquée après entrée d'un antigène dans un organisme, est de deux ordres : c'est soit une réponse immune humorale, soit une réponse immune à médiation cellulaire. La réponse immune humorale, qui comprend l'immunité adaptative à la production d'anticorps, se compose de quatre grandes étapes : la reconnaissance de l'antigène et une sélection clonale, la prolifération clonale, la différenciation des lymphocytes B en plasmocytes qui vont sécréter des immunoglobulines (également appelées anticorps), formant ainsi des complexes immuns pour neutraliser l'antigène. Cette réponse permet d'agir contre les microorganismes extracellulaires. La réponse immune à médiation cellulaire, également appelée réponse immune cellulaire ou réponse CMI (CMI pour Cell-Mediated Immunity), est l'immunité adaptative dans laquelle les lymphocytes T (également appelés cellules T) jouent un rôle central en activant toute une cascade de signaux et réponses faisant face à une infection ou une agression, comme par exemple la production de cytokines. Les cellules effectrices de l'immunité à médiation cellulaire, comme les lymphocytes T cytotoxiques, permettent d'agir contre les microorganismes. Ces deux types de réponses immunitaires « humorales » ou « à médiation cellulaire » mettent en œuvre des cellules sécrétant des molécules, appelées molécules effectrices immunes, à savoir des anticorps ou des cytokines, y compris les chimiokines.

Les antigènes provoquant cette réponse immune peuvent être d'origine infectieuse, exogène, par exemple du fait de pathogènes tels que virus, bactéries, champignons ou parasites, ou du fait des allergènes, ou bien d'origine endogène, par exemple dans le cadre de maladies auto-immunes telles que la sclérose en plaques, le diabète de type 1, le lupus, les thyroïdites auto-immunes, la polyarthrite rhumatoïde, la spondylarthrite ankylosante, le syndrome de Goujerot-Sjôgren, la maladie de Crohn, les maladies cancéreuses, etc.

La multiplication d'un agent pathogène au sein de l'organisme, ou la libération avec des motifs conservés liés au pathogènes ([PAMPs]) tout comme la libération de signaux de danger lors d'un traumatisme, d'une brûlure ou d'une intervention chirurgicale, par exemple, sont responsables d'une réaction immunitaire et inflammatoire. Cette réaction comporte un versant humoral et un versant cellulaire. De plus, elle associe dans l'ensemble de ces situations une réponse pro-inflammatoire (qui est considérée aujourd'hui comme responsable de la survenue de défaillances d'organe et d'une mortalité précoce), ainsi qu'une réponse compensatrice anti-inflammatoire qui, lorsqu'elle persiste, est responsable d'une phase d'immunosuppression, telle qu'on la retrouve chez des patients immunosupprimés, et qui place les patients à risque d'infections secondaires.

En dehors de ces pathologies aiguës, d'autres altérations de la balance immunitaire sont responsables de pathologies. Ainsi, les maladies inflammatoires chroniques soulignent un déséquilibre de la réponse en faveur d'une activation excessive du système immunitaire, avec une destruction chronique des tissus impactés, et la survenue d'une perte de fonction de l'organe touché. A l'opposé, les situations d'immunosuppression innées (déficit immunitaires) ou acquises (infection par le virus du SIDA, chimiothérapie, immunosuppression induite lors d'une transplantation d'organe, par exemple) soulignent une réponse insuffisante du système immunitaire avec un risque infectieux et de formation de tumeur augmenté.

La détection des molécules effectrices produites par des cellules immunes ex vivo permet d'améliorer la prise en charge du patient, que ce soit pour aider au diagnostic de ces pathologies ou pour guider/personnaliser la prise en charge thérapeutique.

Les tests pour détecter la réponse immune à médiation cellulaire (CMI) peuvent être utilisés dans diverses disciplines de l'immunologie. Par exemple, les dosages CMI permettent de détecter les cellules T circulantes réactives au donneur lors d'une transplantation d'organe ; de mesurer l'activité des lymphocytes T cytotoxiques pour la recherche contre le cancer ; de mesurer les réponses-mémoire pour les maladies infectieuses ou le développement de vaccins ; et de détecter les cellules auto-réactives (qui se présentent typiquement à basse fréquence) dans les maladies auto-immunes. Le test ELIspot peut aussi être utilisé en phase préclinique pour déterminer si un produit testé pourrait susciter une réponse immunitaire indésirable, avant de l'utiliser sur des humains.

Le procédé de détection de la réponse cellulaire immune comprend deux phases :
1) Phase 1 - Sécrétion : Incubation des cellules d'intérêt issues d'un échantillon biologique les contenant et susceptibles de sécréter la molécule à détecter, dans des conditions permettant la sécrétion de la molécule. On peut parler alors pour cette étape de « culture cellulaire » ou de « stimulation ». Les cellules sont mises en culture dans un milieu liquide, ce milieu liquide pouvant être soit un fluide biologique (celui du prélèvement), pur ou dilué dans un tampon, soit un milieu de culture, soit un mélange des deux. Cette sécrétion est obtenue en présence d'un stimulant, ce stimulant pouvant être spécifique, comme dans le cas des réponses immunes dirigées contre un agent infectieux, ou pouvant être non spécifique, comme par exemple dans le cas des réponse immunes cellulaires dues à un déficit immunitaire.
2) Phase 2 - Détection de la réponse cellulaire immune : elle peut être mise en œuvre par détection ou quantification de la molécule secrétée à l'issue de la phase 1 (on parle alors plutôt d'analyte) en mettant en œuvre un dosage de la molécule sécrétée, par tout type de technique adaptée à la détection de molécules sécrétées, telle que par immunoessai, par exemple de type ELISA (Enzyme-Linked Immunosorbent Assay), RIA, chimioluminescence ou ELFA (Enzyme-Linked Fluorescent Assay), par bioassay, ou par technique biochimique, par dosage par prolifération, par test de biologie moléculaire ou par cytométrie en flux.

Un exemple de pathologie infectieuse dans laquelle le diagnostic tient compte de la détection de la réponse cellulaire immune due à des cellules de l'organisme infecté est la tuberculose. Pendant plusieurs décennies, le seul test disponible était le test de cuti. Celui-ci permet de savoir si l'individu testé a été en contact avec le bacille *Mycobacterium tuberculosis.* Pour ce faire, le médecin effectue une intra-dermo réaction pour mettre en contact les cellules immunitaires de l'individu avec une préparation de tuberculine. Cette dernière entraine une réponse inflammatoire (cutiréaction) chez les patients ayant été en contact avec le bacille *Mycobacterium tuberculosis* (présence d'une rougeur au niveau de l'injection). Toutefois, la réalisation de la technique est complexe et la lecture du résultat est subjective et nécessite une expertise du soignant. De plus, ce test ne permet pas de quantifier les cytokines sécrétées.

Il existe deux tests sanguins commerciaux qui montrent si les patients ont été en contact avec le bacille *Mycobacterium tuberculosis* en mesurant *in vitro* la libération d'interféron gamma (IFN-y) par les cellules en réponse à des stimulants qui sont des antigènes hautement spécifiques de *Mycobacterium tuberculosis.* Ces antigènes de stimulation sont en particulier au moins ESAT6 et CFP10 et sont absents du vaccin BCG (Bacille Calmette-Guérin) et de la plupart des mycobactéries non-tuberculeuses (Lalvani, A., 2007). Ces tests sont appelés tests IGRA (pour Interferon Gamma Release Assay).

Le premier test est le test T-SPOT^{®}.TB d'Oxford Immunotec [Oxford, Grande-Bretagne], dosage ELISpot sanguin qui mesure la libération de cytokine à partir de cellules isolées. Pour ce test, l'échantillon sanguin est tout d'abord prélevé dans un laboratoire dans un tube stérile hépariné, par exemple un tube Vacutainer^{®} (Becton-Dickinson) ou un tube Greiner, Terumo, Sarstedt, etc. Puis les cellules PBMC (cellules mononucléaires du sang périphérique) sont séparées du sang total par plusieurs étapes de centrifugation sur gradient de densité Ficoll, lavées et comptées. Pour chaque échantillon de sang, les PBMC qui en sont issues sont cultivées dans une plaque de microtitration (appelée aussi microplaque) dans 3 conditions différentes : (i) en présence des antigènes de stimulation spécifiques (par exemple ESAT-6, CFP-10), (ii) en présence d'un ou plusieurs mitogènes non spécifiques (contrôle positif) et (iii) en présence du milieu de stimulation uniquement (contrôle négatif). Les microplaques ainsi préparées sont incubées dans des conditions permettant la sécrétion de l'IFN-y, par exemple à 37°C, 5% CO₂ dans une étuve de culture cellulaire pendant 16 à 20 heures. Cette étape d'introduction des cellules sécrétrices et de culture cellulaire est mise en œuvre sous une hôte à flux laminaire afin de maintenir des conditions stériles pendant la culture cellulaire. La dernière étape consiste à détecter la cytokine ainsi sécrétée à l'aide d'un anticorps de capture anti-IFN-γ préalablement appliqué au fond de la plaque de microtitrage, d'un partenaire de détection se liant à la cytokine et d'un substrat, formant ainsi des spots comptables sur la membrane, chaque spot correspondant à une cellule sécrétrice. Le principe d'un tel dosage ELIspot est décrit par exemple dans l'ouvrage Handbook of Elispot, 2005, et en particulier à la page 17. Ce test a pour inconvénient qu'il est manuel, qu'il nécessite la purification (séparation) au préalable des PBMC à partir du sang total en utilisant une ou plusieurs étapes de centrifugation, ainsi que l'utilisation d'un appareillage particulier (hotte à flux laminaire) afin de garantir la stérilité pour transférer l'échantillon sanguin dans la plaque et pendant la culture cellulaire. De plus, ce test est un test complexe de haute technicité nécessitant un personnel technique bien formé.

Le deuxième test est le test QuantiFERON^{®}-TB Gold [Qiagen] qui mesure la concentration totale en IFN-γ libéré par toutes les cellules dans le surnageant d'un échantillon sanguin par ELISA. Dans ce test, l'échantillon sanguin doit être prélevé chez le patient directement dans trois tubes de prélèvement sanguin stériles spécifiques, l'un contenant les antigènes de stimulation (ESAT-6, CFP-10, et TB7.7), l'autre contenant un mitogène (contrôle positif) et le dernier tube étant le tube dit NIL (contrôle négatif). Puis les tubes, toujours stériles, sont mis à incuber à 37°C pendant 16 à 24 heures pour permettre la sécrétion de l'interféron gamma, de sorte que l'étape de stimulation se déroule en condition stérile. Les tubes sont ensuite centrifugés, le plasma est retiré et placé à l'aide d'une pipette dans une microplaque contenant un anticorps de capture anti-IFN-y. La quantité d'IFN-y est ensuite mesurée par ELISA à l'aide d'un partenaire de révélation et d'un substrat enzymatique. Ce test a plusieurs inconvénients. Tout d'abord ce test n'est pas automatisé. Il nécessite une centrifugation, comme le test T-SPOT^{®}, de sorte qu'il n'est pas facilement automatisable. De plus, comme les tubes de prélèvement sanguin utilisés contiennent déjà les antigènes de stimulation, les cellules sécrétrices d'interféron gamma sont tout de suite mises en contact avec ces antigènes de stimulation, entraînant une manipulation particulière et contraignante jusqu'au dosage de l'IFN-y. En effet, il faut placer ces tubes à la température ambiante (22 +/- 5°C) pour ralentir la sécrétion, et effectuer le dosage d'IFN-y dans un délai donné à partir de la mise en contact du sang et des stimulants (16h maximum). Ceci nuit aussi à la reproductibilité des résultats : en fonction des contraintes d'organisation des laboratoires, le délai entre la collecte de l'échantillon et la stimulation à 37°C pour la sécrétion peut grandement varier. Enfin, comme indiqué précédemment, ce test met en œuvre des tubes de prélèvement stériles particuliers, non seulement contenant au préalable les antigènes de stimulation, mais différents selon l'altitude à laquelle les dosages sont réalisés (références de tube différentes), ce qui est plus difficile d'un point de vue gestion des stocks pour un laboratoire par rapport à l'utilisation de tubes stériles classiques, comme pour le test T-SPOT^{®}.

Bien entendu, la tuberculose n'est pas la seule pathologie pour laquelle on utilise des tests cellulaires, comme souligné précédemment. De plus ces tests sont également utilisés pour d'autres mammifères (Wood P.R., 1990).

Toutefois, quel que soit le test, un inconvénient majeur, outre la difficulté d'automatisation du fait de la nécessité d'une étape de centrifugation, est lié au manque de reproductibilité (Pal M. et al., 2014). Ce manque de reproductibilité est expliqué par plusieurs facteurs dont l'un important concerne le volume d'échantillon qui peut être différent d'un prélèvement sanguin à l'autre. Ceci a pour conséquence de diminuer la sensibilité des tests (Gaur RL, et al., 2013). La fluctuation du volume d'échantillon est due à la prise d'échantillon sanguin réalisée grâce aux tubes utilisés, lesquels sont sous vide, avec une dépression calculée pour prélever environ 1 ml de sang, soit entre 0,8 et 1,2 ml. Ainsi, le ratio antigènes stimulants du tube spécifique/ volume de sang est différent d'un échantillon à un autre, ce qui a forcément un impact sur la quantité de cytokine sécrétée et donc sur la reproductibilité de sa mesure. Pouvoir disposer d'un système qui prélèverait exactement toujours la même quantité d'échantillon permettrait de pallier cet inconvénient.

Almeida CA. M. et al, 2009 proposent un test ELIspot automatisé après la purification des PBMC par centrifugation, comprenant le comptage des cellules et comptage des spots. Pour ce faire, ils utilisent de la robotique de manipulation d'échantillon et réactifs et un système d'information intégré pour suivre électroniquement la performance du dosage. Ce test n'est en fait que partiellement automatisé car l'automatisation n'inclut pas notamment la préparation des PBMC et l'introduction des peptides de stimulation dans les plaques 96 puits. De plus, ce test inclut des étapes particulières, soit avant l'automatisation, telles qu'une étape de centrifugation de l'échantillon pour séparer les cellules, ce qui est difficilement automatisable, soit pendant l'automatisation, telles qu'une étape de comptage des cellules.

Neubauer J.C. et al., 2017 proposent un test ELIspot plus automatisé après la purification des PBMC par centrifugation, allant du comptage des cellules au comptage des spots, en passant par la culture cellulaire (stimulation pour la sécrétion par les cellules). Il s'agit de l'utilisation d'un robot de pipetage de type Tecan, afin d'automatiser les étapes de distribution des cellules, des différents réactifs et des solutions de lavage. Toutefois, cette solution nécessite au moins deux équipements particuliers et chers, ce qui rendrait la fabrication en grande série compliquée. En effet, cette automatisation nécessite notamment l'utilisation d'une hotte à flux laminaire au-dessus de tout le dispositif d'automatisation pour respecter la stérilité lors de la culture cellulaire, condition requise et qui forme la base de la culture cellulaire (Coligan J.E., 1994). Enfin, comme avec le test de Almeida CA. M. et al, 2009, des étapes particulières sont nécessaires, (i) soit avant cette automatisation telles qu'une étape de centrifugation de l'échantillon pour séparer les cellules, ce qui est, comme indiqué précédemment, difficilement automatisable, (ii) soit pendant l'automatisation, telles qu'une étape de comptage des cellules.

La Demanderesse a trouvé contre toute attente qu'il était possible de pallier aux inconvénients des procédés de l'art antérieur et de procéder à la détection de la réponse cellulaire immune en automatisant l'étape de stimulation de cellules qui se fait directement dans l'échantillon biologique à tester tel que prélevé, et donc non modifié après prélèvement, sans avoir recours ni à une étape de centrifugation, ni à une étape de séparation des cellules, ni à une étape de comptage des cellules, ce qui permet de simplifier grandement les instruments automatisés à mettre en œuvre pour une telle détection automatisée.

Aussi, l'invention concerne un procédé de détection d'une réponse cellulaire immune dans un échantillon biologique d'origine animale, susceptible de contenir au moins une cellule sécrétrice d'au moins une molécule effectrice immune, comprenant les étapes consistant à :
(a) aspirer une quantité définie de l'échantillon biologique qui a été préalablement prélevé, par un dispositif automatisé d'aspiration/refoulement,
(b) refouler cette quantité définie, à l'aide dudit dispositif automatisé d'aspiration/refoulement, dans un récipient R, ledit récipient R contenant au préalable au moins un stimulant de sécrétion de ladite molécule par ladite au moins une cellule ou bien ledit récipient R ne contenant pas un tel au moins un stimulant,
(c) lorsque le récipient ne contient pas au préalable au moins un stimulant de sécrétion, introduire dans ledit récipient R à l'aide dudit dispositif automatisé d'aspiration/refoulement, ledit au moins un stimulant de sécrétion contenu dans un récipient E, et
(d) laisser incuber la quantité définie d'échantillon et ledit au moins un stimulant, formant un mélange, pour la sécrétion de ladite molécule par ladite au moins une cellule (i) dans ledit récipient R ou (ii) dans un autre récipient S après aspiration et refoulement dudit mélange à l'aide dudit dispositif automatisé d'aspiration/refoulement, et
(e) détecter la réponse cellulaire immune, dans laquelle la détection de la réponse cellulaire immune indique la présence d'une réponse cellulaire immune spécifique dudit au moins un stimulant de sécrétion, caractérisée en ce que ledit échantillon biologique est un échantillon de sang total qui a été préalablement prélevé chez un organisme animal.

Comme indiqué précédemment, le procédé de détection de la réponse cellulaire immune de l'invention peut être divisé en deux phases : une phase de stimulation des cellules sécrétrices ou susceptibles de sécréter au moins une molécule effectrice immune, contenues dans un échantillon biologique d'origine animale, pour la sécrétion de ladite molécule (étapes (a) à (d)) et une phase de détection de ladite réponse après stimulation (étape (e)). Aussi, l'invention concerne également la première phase, à savoir un procédé automatisé de stimulation d'au moins une cellule susceptible de sécréter une molécule effectrice immune pour la sécrétion de ladite molécule, ladite au moins une cellule étant contenue dans un échantillon biologique de sang total qui a été préalablement prélevé chez un organisme animal, caractérisé en ce qu'il comprend ou consiste en les étapes consistant à :
(a) aspirer une quantité définie de l'échantillon biologique qui a été préalablement prélevé, par un dispositif automatisé d'aspiration/refoulement,
(b) refouler cette quantité définie, à l'aide dudit dispositif automatisé d'aspiration/refoulement, dans un récipient R, ledit récipient R contenant au préalable au moins un stimulant de sécrétion de ladite molécule par ladite au moins une cellule ou bien ledit récipient R ne contenant pas un tel au moins un stimulant,
(c) lorsque le récipient ne contient pas au préalable au moins un stimulant de sécrétion, introduire dans ledit récipient R à l'aide dudit dispositif automatisé d'aspiration/refoulement, ledit au moins un stimulant de sécrétion contenu dans un récipient E, et
(d) laisser incuber la quantité définie d'échantillon et ledit au moins un stimulant, formant un mélange, pour la sécrétion de ladite molécule par ladite au moins une cellule (i) dans ledit récipient R ou (ii) dans un autre récipient S après aspiration et refoulement dudit mélange à l'aide dudit dispositif automatisé d'aspiration/refoulement.

La réponse cellulaire immune ainsi obtenue est particulièrement utile dans diverses applications médicales. Aussi l'invention concerne également l'utilisation du procédé de détection de la réponse cellulaire immune de l'invention ou du procédé automatique de stimulation de l'invention pour le diagnostic de pathologies, pour les études de toxicité de médicaments et vaccins et pour déterminer le statut immunitaire d'un patient.

La Demanderesse a donc trouvé contre toute attente qu'il était possible d'améliorer grandement la détection de la réponse cellulaire immune dans un échantillon biologique d'origine animale susceptible de contenir au moins une cellule sécrétrice d'au moins une molécule effectrice immune en automatisant l'étape de stimulation des cellules directement dans l'échantillon biologique à tester, n'ayant jamais été modifié après le prélèvement, comme par exemple n'ayant jamais subi ni une étape de centrifugation, notamment pour séparer les cellules avant ou après stimulation, ni une étape de comptage des cellules comme dans le cadre d'un dosage par ELIspot et n'ayant jamais été mis en contact avec un stimulant de stimulation. Ceci présente de nombreux avantages dont, entre autres, l'utilisation d'un instrument automatisé simple, la mise en œuvre de tubes standards de prélèvement de l'échantillon biologique, c'est-à-dire ne contenant au préalable aucun stimulant et adapté à toute condition de mise en œuvre, tels que les tubes contenant de l'héparinate de lithium, une meilleure maîtrise du temps total entre la mise en présence de l'échantillon avec le ou les stimulants et la détection de la réponse cellulaire immune, ainsi que la maîtrise du volume d'échantillon pour la stimulation cellulaire.

Par réponse cellulaire immune, on entend tant la réponse immune à médiation cellulaire que la réponse immune humorale générée après stimulation des cellules sécrétrices ou susceptibles de sécréter une molécule effectrice immune avec au moins un stimulant de sécrétion, lesquelles cellules sont contenues dans un échantillon biologique.

Les cellules contenues dans l'échantillon biologique et susceptibles de sécréter au moins une molécule effectrice immune comprennent les cellules effectrices de l'immunité à médiation cellulaire, par exemple les lymphocytes tels que les cellules Natural Killer (NK) et les cellules T (CD4+ et/ou CD8+), lesquelles sécréteront une ou plusieurs cytokines, les cellules effectrices de l'immunité à médiation humorale, par exemple les cellules B, lesquelles sécréteront des anticorps, les macrophages et les monocytes, ainsi que les cellules dendritiques, lesquelles sécrèteront des cytokines ou chimiokines. Comme indiqué précédemment, ces cellules immunes sont capables de sécréter les molécules d'intérêt en présence d'un ou plusieurs stimulants de sécrétion.

Par stimulant de sécrétion, on entend tout élément qui entraine la sécrétion par la cellule, mise en contact avec ce stimulant, d'une molécule effectrice immune qui est une protéine telle que cytokine, anticorps ou marqueur, ou un transcrit ARN du gène de la molécule effectrice immune. Par « transcrit ARN», on entend les ARN, et en particulier les ARN messagers, issus de la transcription du gène de la molécule effectrice immune. Selon la réponse immune que l'on souhaite obtenir, liée à l'état pathologique concerné, lequel est lui-même lié ou non à un microorganisme pathogène, le stimulant sera spécifique du microorganisme pathogène ou non spécifique, ce qui constitue un mode de réalisation particulier de l'invention. Par opposition au stimulant spécifique d'un pathogène, on pourra utiliser indifféremment pour tous les autres stimulants qui sont non spécifiques soit stimulant non spécifique, soit stimulant non spécifique d'un pathogène.

Ainsi, pour les états pathologiques non liés à un microorganisme pathogène, comme par exemple les maladies autoimmunes (par exemple la sclérose en plaques, le diabète de type 1, le lupus, les thyroïdites auto-immunes, la polyarthrite rhumatoïde, la spondylarthrite ankylosante, le syndrome de Goujerot-Sjôgren, la maladie de Crohn), les traumatismes y compris les traumatismes graves comprenant plusieurs traumatismes tels que poly-lésions telles que plaie, fracture, brûlure, etc., les greffes, le procédé de l'invention peut mettre en œuvre un ou plusieurs stimulants non spécifiques, également appelés mitogènes, tels que la protéine kinase A (PKA), le phorbol myristate acétate (PMA) , la phytohemagglutine (PHA), la concanavaline A (conA), le mitogène de Pokeweed (PWM), la Staphylococcal Entérotoxine B (SEB) et le lipopolysaccharide (LPS).

Pour les états pathologiques liés à un microorganisme pathogène, par exemple les états pathologiques liés à un virus tels que le syndrome d'immunodéficience acquise (virus du VIH, SIV, FIV), les hépatites (virus VHC, VHB, VHA, VHE), la varicelle (virus VZV), les infections liées au cytomégalovirus (CMV), la mononucléose infectieuse (virus EBV), les différentes infections type herpès (virus VHS1, VHS2), les états pathologiques liés aux bactéries tels que la tuberculose (*Mycobacterium tuberculosis Mycobacterium bovis, Mycobacterium leprae*), la maladie de Lyme (Borreliose - *Borrelia burgdorferi stricto sensu, Borrelia afzelii, Borrelia garinii, Borrelia spielmanii*), les infections liées aux clostridia (par exemple *Clostridium difficile, Clostridium botulinum*), la salmonelose, les pneumonies et autres pathologies de l'appareil respiratoire (par exemple *Klebsiella, Leugionella*), les maladies urinaires (*Proteus, Klebsiella*), les infections intestinales (par exemple *Escherichia coli, Shigella*), les infections nosocomiales (par exemple *Pseudomonas aeruginosa, Staphylococcus aureus*) ou la Syphilis (*Treponema pallidum),* les états pathologiques liés aux levures telles que les candidoses (*Candida albicans*), les infections fongiques comme les aspergilloses (*Aspergillus fumigatus*) et les mucormycoses (Mucorales), les pathologies liées à un parasite telles que le paludisme (*Plasmodium falciparum, Plasmodium vivax, Plasmodium ovale, Plasmodium malariae, Plasmodium knowlesi*), la schistosomiase (*Schistosomia* mansoni, *Schistosomia* japonicum), le stimulant sera spécifique du pathogène. Il comprendra au moins un antigène spécifique du pathogène entrainant la production par les cellules issues de l'échantillon biologique soit d'une cytokine, soit d'un anticorps dirigé contre cet antigène, en fonction de la nature des cellules stimulées.

Des exemples de stimulants spécifiques du pathogène comprennent les molécules peptidiques (protéines ou parties de protéines) qui peuvent provenir du pathogène, par exemple de l'enveloppe dans le cadre d'un virus, ou elles peuvent être sécrétées par le pathogène lui-même. Ces molécules peuvent être utilisées entières ou sous forme de fragments peptidiques de longueur variable, par exemple d'au moins 7, 8, 9 , 10, 11, 12, 13, 14, 15, 16, 17, 18, 19 ou 20 acides aminés et d'au plus la protéine entière, ou d'au plus 30, 35, 40, 41, 42, 43, 44, 45, 46, 47, 48, 49 ou 50 acides aminés. Le procédé de l'invention peut utiliser un ou plusieurs stimulants peptidiques, de longueur en acides aminés égale ou différente. Par exemple, il peut utiliser au moins un stimulant peptidique de longueur comprise entre 7 et 15 acides aminés et au moins un autre stimulant peptidique d'au moins 16 acides aminés et au plus la protéine entière. Le procédé de l'invention peut utiliser un ou plusieurs stimulants provenant de souches différentes de pathogène.

Lorsqu'un vaccin existe, les stimulants spécifiques utilisés peuvent ne pas être ceux présents dans le vaccin pour permettre ensuite une utilisation du procédé de l'invention dans des applications diagnostiques, par exemple pour vérifier chez le patient vacciné s'il a été en contact avec le pathogène. La composition des vaccins est connue de sorte qu'il est facile pour l'homme du métier de choisir les stimulants antigéniques spécifiques de pathogène appropriés.

Des stimulants spécifiques de pathogène sont bien connus de l'homme du métier. A titre d'exemples non limitatifs, on peut citer le stimulant PPD (Protein Purified Derivate), les protéines ESAT-6, CFP-10, TB7.7, TB37.6, Rv3615c ou leurs fragments pour le pathogène *Mycobacterium tuberculosis* (EP2154248A, EP1144447A, EP2417456A), les protéines pp28, pp50, pp65, IE-1, IE-2, gB ou leurs fragments pour le pathogène Cytomégalovirus et les peptides du virus d'Epstein-Barr (EBV) de type Pep Tivator^{®} fournis par la Société Miltenyi Biotec. On peut également citer les cellules de *Borrelia,* par exemple inactivées par la chaleur, lesquelles peuvent comprendre plusieurs souches de *Borrelia* telles que *Borrelia burgdorferi, Borrelia garinii* et *Borrelia afzelii* ((EP2619584A).

Selon un mode de réalisation, le procédé de l'invention est tel qu'il met en œuvre au moins un stimulant spécifique qui est un antigène hautement spécifique pour *Mycobacterium tuberculosis,* lequel peut être absent du vaccin BCG (Bacille Calmette-Guérin). De préférence, ledit au moins un stimulant spécifique est choisi parmi les antigènes ESAT-6, CFP-10 et TB7.7.

Les cytokines sont toutes cytokines connues sécrétées par les cellules immunes telles que les cellules T, comme par exemple l'interféron gamma (IFN-γ), les interleukines (IL), telles que IL-2, IL-4, IL-6, IL-10, IL-12, IL-13, IL-16, IL-17, IL-la, IL-1β, IL-lra (IL1RN), les chimiokines telles que les chimiokines CC, les chimiokines C, les chimiokines CXC et les chimiokines CX3C, le facteur de nécrose tumorale α (TNFα), le facteur de stimulation de colonies (CSF) tel que le granulocyte (G)-CSF ou le granulocyte macrophage (GM)-CSF, ainsi que le complément ou des composants de la voie biologique du complément, par exemple C5a, Groα (CXCL1), sICAM-1 (CD54), IP-10 (CXCL10), I-TAC (CXCL11), MCP-1 (CCL2), MIF (GIF), MIP-1α (CCL3), MIP-1β (CCL4), RANTES (CCL5) et MIG (CXCL9).

Selon un mode de réalisation, les cellules à stimuler sont des cellules susceptibles de secréter au moins une cytokine.

Selon un autre mode de réalisation, ladite au moins une cytokine qu'on souhaite faire sécréter est choisie parmi les interleukines, les chimiokines, le facteur de nécrose tumorale α (TNFα) et l'interféron gamma (IFNγ), l'interféron gamma étant une cytokine de choix.

Outre les cytokines, les cellules à cultiver selon le procédé automatisé de l'invention peuvent être capables de secréter au moins un anticorps.

Ainsi, selon un mode de réalisation particulier, les cellules cultivées sont des cellules B susceptibles de secréter au moins un anticorps en présence d'au moins un stimulant non spécifique ou d'au moins un stimulant spécifique d'un microorganisme pathogène.

Les stimulants non spécifiques ou spécifiques d'un microorganisme pathogène entraînant la production d'anticorps sont connus de l'homme du métier et des exemples comprennent ceux décrits précédemment pour les cellules T. D'autres exemples comprennent les antigènes de surface des différents pathogènes tels que l'antigène HBs pour le virus de l'hépatite B, l'antigène de surface du virus grippal, ainsi que les protéines d'un individu générant chez lui des autoanticorps. De façon générale, quel que soit le stimulant, celui-ci sera l'antigène spécifique de la réponse anticorps obtenue. Ainsi, l'anticorps qui sera sécrété sera un anticorps dirigé contre ces stimulants antigéniques.

Les échantillons biologiques contenant les cellules susceptibles de sécréter la ou les molécules effectrices immunes appropriés aux fins de l'invention sont des échantillons de sang total prélevés chez un organisme animal.

Les échantillons biologiques ne sont pas modifiés avant leur mise en œuvre dans le procédé de l'invention, c'est-à-dire qu'ils sont utilisés tels quels à la suite du prélèvement chez l'organisme animal et qu'ils ne sont pas mis en contact au préalable avec au moins un stimulant. Ainsi, le prélèvement se fait dans un récipient, par exemple flacon, ne contenant pas de stimulant. De même, et comme indiqué précédemment, les échantillons biologiques ne subissent pas non plus d'étape de séparation ou de comptage des cellules. On peut alors parler d'échantillon brut ou d'échantillon tel que prélevé ou d'échantillon d'origine, cet échantillon étant non préalablement mis en contact avec au moins un stimulant.

Les organismes animaux dont sont issus les échantillons biologiques sont les êtres chez qui on procède habituellement à un diagnostic médical ou à qui on donne des médicaments. Ils comprennent par exemple les mammifères, tels que humains, bovins, canidés, félins, équins, ovins, caprins, porcins, lapins, les reptiliens, les amphibiens, ainsi que les oiseaux tels que gallinacées, ou rapaces.

Selon un mode de réalisation, l'échantillon biologique est un échantillon d'origine humaine, bovine, canine, féline ou équine, de préférence humaine.

La première étape du procédé de détection ou quantification de l'invention comprend l'aspiration d'une quantité définie de l'échantillon biologique qui a été préalablement prélevé, par un dispositif automatisé d'aspiration/refoulement (étape (a)).

Par quantité définie, on entend une quantité ou un volume d'échantillon calibré et toujours identique d'un dosage à un autre du fait de l'utilisation d'un dispositif automatisé d'aspiration/refoulement.

Les dispositifs automatisés d'aspiration/refoulement sont des dispositifs largement connus de l'homme du métier et sont présents dans un grand nombre d'instruments de biochimie, de biologie moléculaire et d'immunoessai, par exemple les instruments de la gamme VIDAS^{®} comme le VIDAS^{®}, miniVIDAS^{®}, VIDAS^{®} 3 (bioMérieux), le Simoa^{®} HD-1 (Quanterix), le Cobas^{®} ou l'Elecsys^{®} (Roche Diagnostic Gmbh), le LIAISON^{®} (Diasorin), l'Architect^{®} (Abbott), l'Access 2 (Beckman Coulter), le Clarity^{™} (Singulex^{®}), le Vitros^{®} (Johnson & Johnson), le Centaur^{®} (Siemens), l'eMAG^{®} et l'easyMAG^{®} (bioMérieux). Ces dispositifs automatisés d'aspiration/refoulement comprennent un ou plusieurs dispositifs de prélèvement permettant d'aspirer un milieu liquide, tel que l'échantillon biologique ou les milieux réactionnels, à partir du récipient dans lequel il se trouve, puis ensuite de le refouler. Ils comprennent par exemple un bras ou un support au bout duquel est inséré une aiguille ou un embout (tip ou cône), lequel embout est vierge ou préalablement revêtu de partenaires de liaison, comme par exemple le SPR^{®} de la gamme VIDAS^{®}. Comme le dispositif de prélèvement sert à aspirer puis à refouler différents milieux, tout d'abord l'échantillon biologique, puis divers milieux réactionnels, les embouts dudit dispositif d'aspiration/refoulement peuvent être changés et rejetés dans une poubelle à divers moments du procédé. Ainsi, par exemple, dans le cadre de l'instrument VIDAS^{®}, un premier embout est utilisé pour prélever l'échantillon biologique, puis si nécessaire, un autre embout est utilisé pour apporter ledit au moins un stimulant de sécrétion, puis un autre embout revêtu de partenaire de liaison (SPR^{®}) est utilisé pour la mesure de la réponse cellulaire. Lorsque le dispositif d'aspiration/refoulement comporte plusieurs dispositifs de prélèvement, chaque dispositif de prélèvement possède son rôle propre. Par exemple, un premier dispositif de prélèvement peut servir à aspirer/refouler l'échantillon biologique et un deuxième dispositif de prélèvement peut servir à aspirer/refouler les réactifs nécessaires par exemple à l'incubation et à la mesure de la réponse cellulaire. Le dispositif de prélèvement est guidé par un dispositif de commande d'aspiration/refoulement, comme par exemple un dispositif effectuant une différence de pression dans le dispositif de prélèvement à l'aide d'un piston entrainé par tout moyen connu de l'homme du métier, comme par exemple à l'aide d'une vis couplée à un moteur, formant un bloc pompe, le dispositif de prélèvement et le dispositif de commande d'aspiration/refoulement pouvant être reliés par l'intermédiaire d'une tubulure comportant des joints pour garantir l'étanchéité. Le dispositif de prélèvement et le dispositif de commande d'aspiration/refoulement peuvent être plus ou moins éloignés. L'homme du métier adaptera la vitesse et la puissance d'aspiration du dispositif de commande d'aspiration/refoulement en fonction de la quantité d'échantillon que l'on souhaite prélever en vue de la stimulation et, le cas échéant, en fonction de la longueur et section de la tubulure entre le dispositif de commande d'aspiration/refoulement et le récipient échantillon. Ainsi, par exemple, dans le cadre de l'instrument VIDAS^{®} 3 où le bloc pompe est proche du cône, la vitesse d'aspiration sera comprise entre 10 et 500 µl/seconde.

Le récipient échantillon est tout récipient adapté pour recevoir un échantillon : tube, flacon. Il peut être en matière plastique, telle qu'en éthylene acétate de vinyle (copolymère EVA), Polyéthylène, Polypropylène copolymère, Fluorinated ethylene propylene (nalgène), en verre ou en inox. Dans le cas du sang ou dérivé, le récipient échantillon pourra être un tube de prélèvement sanguin type sous vide, par exemple un tube Vacutainer^{®}, Greiner ou Terumo, par exemple un tube hépariné, utilisé classiquement pour prélever du sang chez un patient.

Le récipient échantillon ou son contenu sera placé dans l'instrument mettant en œuvre le procédé automatisé de l'invention avant la mise en œuvre de ce procédé, par exemple par un opérateur ou par un autre dispositif automatisé.

La quantité/volume d'échantillon biologique qui sera aspirée par ledit dispositif automatisé d'aspiration/refoulement sera définie facilement par l'homme du métier en fonction de plusieurs paramètres tels que l'instrument qu'il va utiliser et la nature de l'échantillon biologique. De façon générale, la quantité d'échantillon biologique qui sera aspirée à partir du récipient échantillon sera comprise entre 5 et 800 µL, de préférence entre 10 et 500 µL. Cette quantité sera d'au moins 10, 15, 20, 25, 30, 35 ou 40 µL et d'au plus 100, 150, 200, 250, 300, 350 ou 400 µL.

Une fois la quantité définie calibrée d'échantillon biologique aspirée, celle-ci est ensuite refoulée par le même dispositif d'aspiration/refoulement dans un récipient appelé récipient R dans lequel la stimulation proprement dite (incubation) va pouvoir se faire.

Comme pour le récipient échantillon, le récipient R peut être en matière plastique, telle qu'en éthylene acétate de vinyle (copolymère EVA), Polyéthylène, Polypropylène copolymère, Fluorinated ethylene propylene (nalgène), en verre ou en inox.

Le récipient R peut contenir au préalable tous les composants nécessaires à la stimulation, par exemple le cas échéant ledit au moins un stimulant de sécrétion, ainsi qu'un ou plusieurs tampons tels que les tampons phosphate, borate, Tris, HEPES, ou bien il peut contenir uniquement certains des composants, par exemple le cas échéant ledit au moins un stimulant de sécrétion, notamment sous forme lyophilisée (en poudre, microbilles, capsules, etc.), les autres composants, présents dans un autre récipient, étant apportés dans le récipient R par le dispositif d'aspiration/refoulement après refoulement de la quantité définie d'échantillon biologique pour la mise en œuvre de l'étape d'incubation. Ou bien encore le récipient R peut être vide et les composants nécessaires à l'incubation, présents dans un autre récipient, étant apportés dans le récipient R par le dispositif d'aspiration/refoulement après refoulement de la quantité définie d'échantillon biologique pour la mise en œuvre de l'étape d'incubation. Notamment ledit au moins un stimulant de sécrétion peut être contenu dans un récipient E et est introduit dans le récipient R par le dispositif d'aspiration/refoulement après refoulement de la quantité définie d'échantillon biologique (étape (c)). Ledit au moins un stimulant de sécrétion est contenu dans le récipient E soit sous forme lyophilisée, soit en mélange avec un ou plusieurs tampons, comme décrit précédemment. Selon un mode de réalisation, le récipient R ne contient pas ledit au moins un stimulant de sécrétion qui est contenu dans un récipient E.

Ce récipient R ou E peut être constitué d'un seul élément unitaire, par exemple un flacon, un tube, une boîte ou un puits, ou bien il peut être inclus dans un ensemble de plusieurs récipients, comme par exemple une plaque de microtitration ou une barrette contenant plusieurs puits, telle que la barrette vendue pour sa mise en œuvre dans l'instrument VIDAS^{®} (bioMérieux, France). Dans ce dernier cas, les récipients R et E pourront être dans la même plaque ou la même barrette.

Le récipient R est stérile ou non avant l'introduction de la quantité définie d'échantillon biologique. En tout état de cause, il est propre, c'est-à-dire qu'il contient une quantité de germe très faible. Le récipient R peut être recouvert par un élément obturateur qui sera percé pour l'introduction de la quantité définie d'échantillon. Par exemple, le récipient R sera percé par le dispositif d'aspiration/refoulement lors de l'étape de refoulement. Cela s'applique également au récipient E. L'élément obturateur est par exemple une feuille d'aluminium, un film polymère, un septum ou une feuille plastique collée. L'ouverture de l'élément obturateur pourra varier de partiellement à totalement, c'est-à-dire que le récipient de stimulation R pourra être entièrement ouvert ou ouvert uniquement sur le diamètre du dispositif d'aspiration/refoulement. Par exemple, si le dispositif de prélèvement contient un embout de pipette, lequel a une forme conique à son extrémité, le dispositif d'aspiration/refoulement sera programmé par son dispositif de commande d'aspiration/refoulement pour que l'élément obturateur ne soit percé que par la partie comprenant le diamètre le plus petit de l'embout. Ainsi, l'élément obturateur peut être percé sur un diamètre compris entre 0,5 mm et 5 mm.

L'incubation de la quantité définie d'échantillon et dudit au moins un stimulant, formant un mélange échantillon-stimulant, également appelée étape de stimulation, peut être mise en œuvre à une température à laquelle les cellules se trouvent habituellement *in vivo,* à savoir à une température proche de la température centrale moyenne de l'organisme animal dont est issu l'échantillon biologique, ce qui constitue un mode de réalisation particulier de l'invention. En effet, chaque organisme possède une température moyenne centrale qui lui est propre. Ainsi la température moyenne centrale pour un homme est de 37°C, celle des bovins de 38,6°C, celle des chiens de 38,9°C, celles des chats de 38,6°C, celle des chevaux de 37,8°C, celle des lapins de 39,5°C et celle des poulets de 41,7°C. Pour les vertébrés à sang froid, la température moyenne centrale est comprise entre 18° à 25°C. Ainsi, selon que l'échantillon biologique provient d'un organisme animal ou d'un autre, la température à mettre en œuvre dans l'étape d'incubation du procédé de l'invention pourra être différente et l'homme du métier choisira la bonne température en fonction de l'échantillon biologique utilisé et de la température moyenne centrale correspondante. Comme les cellules sont capables de supporter une variation, la température d'incubation dans le procédé de l'invention sera de préférence celle de la température centrale de l'organisme animal dont est issu l'échantillon biologique + ou - 3°C, de préférence + ou - 2°C, ou + ou - 1°C. Par exemple, pour l'être humain, les cellules seront stimulées entre 34 et 40°C, de préférence entre 35 et 39°C, ou encore entre 36 et 38°C, ou encore à 37°C. De façon générale, la température d'incubation sera comprise entre 18 et 45°C, de préférence entre 35°C et 43°C. Selon un mode de réalisation, la température de l'étape d'incubation sera d'au moins 19, 20, 21, 22, 23, 24, 25, 26, 27, 28, 29, 30, 31, 32, 33, 34 ou 35°C. La température pourra être d'au plus 38, 39, 40, 41, 42, 43 ou 44°C.

La durée de l'étape d'incubation peut varier, notamment en fonction de la quantité de cellules présentes dans l'échantillon biologique, et est comprise de façon générale entre 1h et 72h. Selon un mode de réalisation, la durée de l'étape d'incubation est d'au moins 2, 3, 4, 5, 6, 7, 8, 9, 10, 11, 12, 13, 14, 15, 16, 17 et 18 h et elle est d'au plus 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 51, 54, 57, 60, 63, 66, 69, 70 ou 71 h. De préférence, la durée de l'étape d'incubation est comprise entre 2 et 24h, et de préférence encore entre 6 et 18h.

L'incubation peut se dérouler (i) dans ledit récipient R, ce qui est préféré, ou bien (ii) dans un autre récipient S après aspiration et refoulement dudit mélange à l'aide dudit dispositif automatisé d'aspiration/refoulement.

Il est classiquement connu de procéder à l'étape d'incubation des cellules immunes, contenues dans l'échantillon biologique ou séparées, comme en Elispot, avec le ou les stimulants de sécrétion en condition stérile. Or la Demanderesse a montré que cette condition n'était pas nécessaire. Aussi, le récipient R n'a pas besoin d'être mis en condition stérile pendant l'étape d'incubation des cellules. Ainsi, selon un mode de réalisation, le récipient R est non stérile après introduction de la quantité définie d'échantillon et/ou l'étape d'incubation (d) se déroule en condition non stérile, ce qui est tout à fait inattendu.

Si la mise en œuvre d'une étape d'incubation en condition stérile nécessite un dispositif particulier pour respecter cette condition, comme la hotte à flux laminaire ou le tube de prélèvement de sang sous vide, la mise en œuvre d'une étape d'incubation en condition non stérile est particulièrement facile dans la mesure où elle ne nécessite aucun dispositif particulier. Ainsi, tout instrument de biochimie, biologie moléculaire ou immunoessai comprenant un dispositif automatisé d'aspiration/refoulement tel que décrit précédemment est adapté pour une telle étape d'incubation en condition non stérile.

La dernière étape du procédé de l'invention consiste à détecter la réponse cellulaire immune qui est spécifique dudit au moins un stimulant de sécrétion, et ce directement à partir du mélange échantillon-stimulant puisque, contre toute attente, aucune centrifugation des cellules n'est nécessaire avant la détection de cette réponse cellulaire immune. Par spécifique dudit au moins un stimulant de sécrétion, on entend que l'utilisation d'un autre stimulant avec le même type de cellule (B ou T) pourrait donner une réponse différente, par exemple d'autres anticorps ou d'autres cytokines.

La détection de la réponse cellulaire immune peut être mise en œuvre par toute méthode connue de l'homme du métier telle que par immunoessai, par dosage par prolifération, par mesure du niveau d'expression des transcrits ARN selon les méthodes connues en biologie moléculaire ou par cytométrie en flux. Elle mettra ou non en œuvre au moins un partenaire de liaison à la molécule effectrice immune ou à son transcrit ARN correspondant, lequel comprend tout partenaire capable de se lier à ladite molécule ou audit transcrit. La nature du partenaire de liaison dépendra de la nature de l'analyte et du type d'essai mis en œuvre. De préférence, le test sera un immunoessai ou un test de biologie moléculaire.

Bien entendu, le terme « immuno » dans « immunoessai » par exemple, n'est pas à considérer dans la présente demande comme indiquant strictement que le partenaire de liaison est un partenaire immunologique, tel qu'un anticorps. En effet, l'homme du métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (« Enzyme-Linked Immunosorbent Assay » littéralement « dosage d'immunoadsorption par enzyme liée ») pour des dosages qui utilisent des partenaires de liaison non immunologiques, appelés plus largement en anglais « Ligand Binding Assay », que l'on pourrait traduire par « Dosage utilisant la liaison à un ligand », alors que le terme « immuno » est inclus dans l'acronyme ELISA. Par souci de clarté, la Demanderesse utilisera dans toute la demande le terme « immuno » pour tout test ou dosage utilisant un partenaire de liaison, même quand il n'est pas un partenaire immunologique.

Si l'étape (e) met en œuvre au moins œuvre au moins un partenaire de liaison à la molécule effectrice immune, elle peut comprendre les étapes consistant à :
(e') mettre en contact ladite molécule effectrice immune sécrétée avec au moins un partenaire de liaison à ladite molécule et
(f') révéler la présence ou la quantité de molécule par sa liaison audit au moins un partenaire de liaison.

A titre d'exemples de partenaire de liaison d'immunoessai, on peut citer les partenaires de liaison de nature ou d'origine immunologique tels que les anticorps (monoclonaux ou polyclonaux) et les fragments d'anticorps (tels que Fab, Fab', F(ab')2), chaînes scFv (Single chain fragment variable), dsFv (Double-stranded fragment variable)), bien connus de l'Homme du Métier, ainsi que les partenaires de liaison n'étant pas de nature ou d'origine immunologique tels que les protéines différentes des anticorps, les peptides, les oligonucléotides, les nanofitines, les récepteurs à la molécule d'intérêt s'ils existent, les aptamères, les DARPins ou toute autre molécule qui est connue pour avoir une interaction avec ladite molécule d'intérêt.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les aptamères sont des oligonucléotides, généralement ARN ou ADN, identifiés dans des banques contenant jusqu'à 1015 séquences différentes, par une méthode combinatoire de sélection in vitro appelée SELEX pour « Systematic Evolution of Ligands by Exponentiel Enrichment » (Ellington AD et Szostak JW., 1990). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celles des anticorps.

Les « DARPins » pour Designed Ankyrin Repeat ProteINS (Boersma YL et Plütckthun A, 2011) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par « screening » de banques combinatoires.

Des exemples de partenaires de liaison comprennent les anticorps anti-IFN-γ, les anticorps anti-anticorps, les protéines de pathogène ou leurs fragments (par exemple la protéine NS3 du VHC, HBsAg du VHB, etc.) et les protéines de phages, lesquels sont largement connus de l'homme du métier et disponibles dans le commerce.

La mise en contact de la molécule effectrice immune sécrétée et dudit au moins un partenaire de liaison est mise en œuvre dans un récipient différent du récipient R ou dans le récipient R. Dans ce dernier cas, le partenaire de liaison est contenu au préalable dans le récipient R ou bien il y sera apporté ultérieurement par le dispositif d'aspiration/refoulement. Si la mise en contact de la molécule effectrice immune sécrétée et dudit au moins un partenaire de liaison est mise en œuvre dans un récipient différent du récipient R, par exemple dans un récipient T, selon un mode de réalisation, c'est le contenu du récipient R qui sera aspiré puis refoulé dans le récipient T à l'aide du dispositif d'aspiration/refoulement. Là-encore, le partenaire de liaison peut être contenu au préalable dans le récipient T, ou bien il y sera apporté ultérieurement par le dispositif d'aspiration/refoulement. Ledit au moins un partenaire de liaison peut être soit immobilisé à la surface des récipients (R ou T), ou bien il peut y être présent sous forme lyophilisée. Selon un autre mode de réalisation, le partenaire de liaison est immobilisé à la surface d'un embout utilisé avec le dispositif d'aspiration/refoulement comme par exemple dans le cadre des kits mis en œuvre avec l'instrument VIDAS^{®} (cône SPR^{®}). L'application d'un tel partenaire à la surface d'un récipient ou embout est largement connue de l'homme du métier.

Les méthodes d'immunoessai sont largement connues de l'homme du métier et comprennent par exemple les dosages immuno-enzymatique ou EIA pour « Enzyme Linked Immunoassay ». Celles-ci sont couplées à une réaction catalysée par une enzyme en utilisant un substrat enzymatique. Selon le substrat enzymatique choisi, on peut avoir un signal colorimétrique (ELISA pour Enzyme-Linked Immunosorbent Assay) (Rassasie, M.J., et al., 1992), un signal de fluorescence (technologie ELFA pour Enzyme Linked Fluorescent Assay) ou un signal chemiluminescent (CLIA pour Chemiluminescence Immuno Assays) (Stabler T.V., et al., 1991).

La détection de la réponse cellulaire immune peut être qualitative, semi-quantitative ou quantitative. Dans le cadre des immunoessais, elle est basée sur des mesures permettant de quantifier les signaux émis au cours de l'analyse de l'échantillon biologique contenant la molécule d'intérêt. La quantité de signaux détectée est généralement proportionnelle à la quantité de molécule d'intérêt à mesurer (par exemple lors d'un essai de type sandwich, utilisant deux partenaires de liaison) ou inversement proportionnelle à la quantité d'analyte à mesurer (par exemple lors d'un essai en compétition, utilisant un seul partenaire de liaison et un composé qui entre en compétition avec la molécule d'intérêt).

Des étapes classiques pour un immunoessai de type sandwich dans un échantillon biologique, également appelé échantillon de test, susceptible de contenir ledit analyte ou molécule d'intérêt, comprennent ou consistent en :
- la mise en présence dudit au moins un partenaire de liaison et dudit échantillon de test susceptible de contenir la molécule d'intérêt pour la fixation de la molécule d'intérêt sur le partenaire de liaison,
- l'ajout d'un partenaire de détection, lequel est couplé directement ou indirectement à un marqueur, telle qu'une enzyme capable de lyser un substrat enzymatique, par exemple fluorogène pour une détection ELFA, pour sa fixation sur le complexe partenaire de liaison-molécule d'intérêt,
- lorsque le marqueur est une enzyme, la mise en présence d'un substrat enzymatique et du complexe partenaire de liaison-molécule d'intérêt-partenaire de détection couplé à une enzyme pour la formation d'un milieu réactionnel, et
- la détection, par exemple par immunofluorescence dans le cadre d'une détection ELFA, de la présence et/ou la quantité de molécule d'intérêt en mesurant le signal (par exemple fluorescence) émis dans le milieu réactionnel.

Par partenaire de détection, on entend tout partenaire capable de se lier à la molécule d'intérêt à détecter ou quantifier, lequel sera couplé directement ou indirectement à un marqueur, par exemple une enzyme. Il peut être de même nature que le partenaire de liaison ou de nature différente. Des exemples sont donnés précédemment avec le partenaire de liaison.

Par couplage direct ou indirect du marqueur sur le partenaire de détection, on entend que le marqueur est fixé directement sur le partenaire de détection reconnaissant l'analyte (couplage direct) ou bien l'enzyme est couplé à un partenaire de liaison qui reconnaît le partenaire de détection qui reconnaît lui-même l'analyte (couplage indirect).

Ainsi, dans le cadre du couplage direct, le complexe formé à la fin du dosage, appelé conjugué, sera constitué de : « Partenaire de liaison/molécule d'intérêt/partenaire de détection couplé au marqueur ».

Dans le cadre du couplage indirect, le complexe formé à la fin du dosage sera constitué de : « Partenaire de liaison/molécule d'intérêt/partenaire de détection/partenaire de liaison couplé au marqueur ».

Par marqueur, on entend, notamment, toute molécule contenant un groupement réactif avec un groupement du partenaire de détection, directement sans modification chimique, ou après modification chimique pour inclure un tel groupement, laquelle molécule est capable de générer directement ou indirectement un signal détectable. Une liste non limitative de ces marqueurs de détection directe consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Bien entendu, le partenaire de liaison peut être lui-même marqué comme indiqué précédemment. Dans le cadre d'un test sandwich, le deuxième partenaire utilisé ne sera alors pas marqué.

Ainsi, selon un mode de réalisation, le procédé de détection de l'invention est caractérisé en ce que l'étape (e) met en œuvre un partenaire de liaison à la molécule effectrice immune qui est un partenaire d'immunoessai et en ce que la révélation de la liaison ou non de ladite molécule est mise en œuvre par un test de type sandwich utilisant un autre partenaire de liaison à la molécule, de nature différente ou non, l'un des deux partenaires étant marqué.

Des étapes classiques pour un immunoessai de type compétition, dans un échantillon de test susceptible de contenir ladite molécule d'intérêt, comprennent :
- la mise en présence ledit au moins un partenaire de liaison, d'un analogue à la molécule d'intérêt couplé à un marqueur, par exemple une enzyme capable de lyser un substrat enzymatique, par exemple fluorogène, et dudit échantillon, lesquels entrent en compétition pour la fixation sur le partenaire de liaison,
- lorsque le marqueur est une enzyme, la mise en présence d'un substrat enzymatique, des complexes partenaire de liaison-molécule d'intérêt et partenaire de liaison-analogue à la molécule d'intérêt pour la formation d'un milieu réactionnel, et
- la détection, par exemple par immuno fluorescence, de la présence et/ou quantité d'analyte en mesurant le signal, par exemple la fluorescence, émis dans le milieu réactionnel.

Par analogue à l'analyte, on entend toute molécule qui présente les mêmes capacités de liaison au partenaire de liaison que la molécule d'intérêt.

Le marqueur couplé à l'analogue à la molécule d'intérêt est équivalent au marqueur utile dans le cadre d'un test sandwich.

Selon un autre mode de réalisation particulier de l'invention, le procédé de détection de l'invention est caractérisé en ce que l'étape (e) met en œuvre un partenaire de liaison à la molécule effectrice immune qui est un partenaire d'immunoessai et en ce que la révélation de la liaison ou non de ladite molécule est mise en œuvre par un test en compétition utilisant un composé marqué entrant en compétition avec la molécule d'intérêt.

Quel que soit le type de méthode utilisée, de type sandwich ou en compétition, l'enzyme est un marqueur largement approprié et on peut citer notamment comme exemple la sulfatase, la peroxidase, la phosphatase alcaline (PAL), la phosphatase acide, la glucose oxydase (GOx), la glucose-6-phosphate déshydrogénase (G6PD) et la β-galactosidase (β-gal). Les substrats enzymatiques correspondants sont largement connus de l'homme du métier et comprennent par exemple le 4-méthylombelliferyl phosphate ou le 5-bromo-4-chloro-3-indolyl-beta-D-galactopyranoside.

Selon un mode de réalisation particulier, dans le procédé de détection selon l'invention, la révélation de l'analyte est mise en œuvre en utilisant une enzyme et un substrat enzymatique catalysé par ladite enzyme, de préférence la phosphatase alcaline et le 4-méthylombelliferylphosphate.

Lorsque la réponse cellulaire immune est obtenue par détection du transcrit d'ARN du gène de la molécule effectrice immune, appelé également gène cible, la mesure de son niveau d'expression met en œuvre des méthodes de détection d'ARN bien connues de l'homme du métier, à l'aide d'instruments classiques tels que le Filmarray^{®} (bioMérieux), LightCycler1.0, 2.0 et 480 (System II), Applied Biosystems 7500, 7500 Fast, 7500 Fast Dx, 7300, ViiA^{™} 7 Real-Time PCR System, StepOne, Rotor-Gene, Dx Real-Time System et CFX 96 Real-Time System.

Ainsi, ces méthodes peuvent tout d'abord comprendre une étape préliminaire d'extraction des ARN totaux de l'échantillon biologique (ARN ribosomaux, ARN de transfert et ARN messagers). Cette étape est suivie d'une étape de transcription inverse de ces différents ARN afin d'obtenir leur ADN complémentaire (ADNc). Ensuite les ADNc spécifiques du gène cible sont amplifiés, détectés puis quantifiés.

L'extraction est mise en œuvre par tous les protocoles d'extraction et de purification d'acides nucléiques bien connus de l'homme du métier. A titre indicatif, l'extraction d'acides nucléiques peut être réalisée par lyse des cellules présentes dans l'échantillon biologique suivie d'une purification, par exemple sur des billes magnétiques, ou encore par extraction par du phénol, du chloroforme et de l'alcool. Ces étapes sont bien connues de l'homme du métier et sont décrites par exemple dans les brevets US5234809, EP0245945, WO93/01312, WO00/21973, WO02/16383 ou dans les notices techniques des kits et plateformes de préparation d'échantillons (sample prep) MagNa Pure, QiaCube, EasyMAG^{®}, QiaAmp DNA mini Kit.

On réalise ensuite une réaction de transcription inverse à l'aide d'une enzyme transcriptase inverse qui permet d'obtenir, à partir d'un fragment d'ARN, un fragment complémentaire d'ADN (ADNc). La réalisation d'une telle étape est bien connue de l'homme du métier (revue générale, 2003). Lorsque l'on souhaite plus particulièrement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription inverse réalisée par l'enzyme transcriptase inverse. On obtient alors différents ADN complémentaires des différents ARN messagers initialement présents dans l'échantillon biologique.

Ensuite, dans le but d'amplifier spécifiquement les ADNc spécifiques du gène cible, une réaction d'amplification enzymatique est réalisée. Une réaction d'amplification enzymatique est un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US4683195, US4683202 et US4800159, et toutes ses variantes ;
- LCR (Ligase Chain Reaction), exposée par exemple dans la demande de brevet EP0201184 ;
- RCR (Repair Chain Reaction), décrite dans la demande de brevet WO90/01069 ;
- 3SR (Self Sustained Sequence Replication) avec la demande de brevet WO90/06995 ;
- NASBA (Nucleic Acid Sequence-Based Amplification) avec la demande de brevet WO91/02818 ;
- TMA (Transcription Mediated Amplification) avec le brevet US5399491 ;
- LAMP (Loop mediated isothermal amplification) avec le brevet US6410278 ;
- SDA (Strand Displacement Amplification) décrite dans Y. Masamute et C.C. Richardson, 1971, J. Biol. Chem. 246, 2692-2701 ; R.L. Lechner et al., 1983, J. Biol. Chem. 258, 11174-11184 ; ou R.C. Lundquist et B.M. Olivera, 1982, Cell 31, 53-60;
- CRCA (cascade rolling circle amplification) ;
- ICAN (isothermal and chimeric primer-initiated amplification of nucleic acids) décrite dans T Uemori, 2007, J Biochem. 2007 Aug;142(2):283-92 ;
- HDA (target based-helicase dépendent amplification) décrite dans Vincent et al., 2004, Embo reports, 5(8) : 795-800.

On parle alors d'amplicons pour désigner les polynucléotides générés par une technique d'amplification enzymatique. Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène cible. Lorsque l'amplification enzymatique est une PCR réalisée après une réaction de transcription inverse, on parle de RT-PCR. Parmi les variantes de la PCR, on peut citer notamment la réaction en chaîne par polymérase emboitée ou PCR nichée (Nested-PCR) qui implique deux amplifications PCR grâce à la présence de deux PCR successives et deux paires d'amorces différentes : la première paire d'amorces sert pour la première PCR (PCR classique), la seconde paire d'amorces servant lors de la seconde PCR et étant définie de telle sorte qu'elle s'hybride sur une séquence présente dans le produit d'amplification (amplicon) de la première PCR.

Suite à cette étape d'amplification, quelle que soit la technique utilisée, le niveau d'expression du gène cible est déterminé par exemple par hybridation à l'aide d'au moins une sonde d'hybridation spécifique du produit d'expression de ce gène cible, qui correspond au partenaire de liaison au transcrit ARN d'intérêt, ou à l'aide d'un intercalant de l'ADN double brin tel que le SybrGreen^{®}.

Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec un fragment nucléotidique cible. Dans la présente invention, le fragment nucléotidique cible peut être une séquence nucléotidique comprise dans un ARN messager ou une séquence nucléotidique comprise dans un ADN complémentaire obtenu par transcription inverse dudit ARN messager.

Les techniques d'hybridation sont bien connues de l'homme du métier, et on peut citer notamment la technique du Northern blot.

La quantification des ARNm, produits d'expression du gène cible, passe par une étape de détection de la réaction d'hybridation, entre la sonde d'hybridation ou l'intercalant de l'ADN et le fragment nucléotidique cible.

Par détection, on entend soit une détection directe par une méthode physique, soit une méthode de détection à l'aide d'un marqueur. De nombreuses méthodes de détection existent pour la détection des acides nucléiques (Keller G.H., 1993; Kricka, 1999).

L'homme du métier pourra se référer notamment aux publications de Bustin SA et Giulietti A *et al.* (Bustin SA, 2002 ; Giulietti A, 2001).

Tout ce qui a été décrit précédemment sur les immunoessais s'agissant des différentes étapes de procédé et de l'utilisation d'un partenaire de liaison peut s'appliquer à la détection par biologie moléculaire.

Le procédé de détection de l'invention peut également comprendre une ou plusieurs étapes supplémentaires de rinçage après chaque étape, comme par exemple :
- avant l'ajout du partenaire de détection, une étape de rinçage de façon à éliminer la molécule non liée au complexe partenaire de liaison-molécule d'intérêt ; et
- après l'ajout du partenaire de détection, une étape de rinçage de façon à éliminer le partenaire de détection non lié.

Les étapes de rinçage sont des étapes connues de l'homme du métier. Elles sont mises en œuvre avec des tampons compatibles avec le milieu réactionnel et la lecture du signal.

Les étapes (a) à (d) sont bien entendu mises en œuvre avant l'étape (e), mais plusieurs modes de réalisation sont possibles. Ainsi, le procédé de détection de l'invention comprend une ou plusieurs des caractéristiques suivantes :
- les étapes (a) à (e) sont mises en œuvre avec le même instrument ;
- les étapes (a) à (e) sont mises en œuvre successivement dans la même journée ;
- les étapes (a) à (d) sont mises en œuvre un premier jour ou pendant plusieurs jours, par exemple jusqu'à 3 jours, comme défini précédemment, et l'étape (e) est mise en œuvre ensuite un autre jour, par exemple 1, 2 ou 3 jours plus tard.

Par même instrument, on entend le même instrument pour faire toutes les étapes successivement, ou bien deux instruments de la même gamme, l'un pour mettre en présence ledit au moins un stimulant et l'échantillon biologique tel que prélevé, et l'autre pour détecter la réponse cellulaire immune. Ainsi, grâce à l'utilisation du même instrument, le procédé est complètement automatisé en utilisant un équipement simplifié ne nécessitant ni la présence d'un personnel hautement qualifié, ni la mise en œuvre de conditions très spécifiques lorsque l'étape d'incubation est mise en œuvre en condition non stérile. Dans ce mode de réalisation particulier, le dispositif d'aspiration/refoulement pourra être utilisé pour transférer l'échantillon biologique contenant la molécule d'intérêt sécrétée vers le ou les récipients utilisés pour l'étape (e), comme indiqué précédemment. Le ou les différents récipients nécessaires à la mise en œuvre de toutes les étapes du procédé de l'invention pourront être mis dans l'instrument avant l'étape (a) ou bien ils pourront être mis en deux temps, en fonction de la phase (stimulation/détection).

Les exemples d'instruments pouvant être utilisés comprennent tous les instruments de diagnostic déjà disponibles dans le commerce, par exemple ceux d'immunoessai ou de biologie moléculaire, lesquels comprennent :
- Un dispositif d'aspiration/refoulement pour prélever l'échantillon biologique issu d'un patient et le mettre dans un récipient pour la stimulation, puis ensuite pour la détection de la réponse cellulaire immune,
- Un ensemble de récipients pour pouvoir réaliser la stimulation et la détection,
- Optionnellement un système de maintien de la température du récipient servant à l'incubation à la température choisie et
- Un système de détection de la réponse cellulaire immune.

Des exemples non limitatifs d'instruments d'immunoessai adaptés comprennent les instruments de la gamme VIDAS^{®} comme le VIDAS^{®}, miniVIDAS^{®}, VIDAS^{®} 3 (bioMérieux), le Simoa^{®} HD-1 (Quanterix), le Cobas^{®} ou l'Elecsys^{®} (Roche Diagnostic Gmbh), le LIAISON^{®} (Diasorin), l'Architect^{®} (Abbott), l'Access 2 (Beckman Coulter), le Clarity^{™} (Singulex^{®}) et le Vitros^{®} (Johnson & Johnson).

Par exemple, l'instrument de la gamme VIDAS^{®} (bioMérieux) utilise un dispositif d'aspiration/refoulement consistant en un cône (SPR^{®}) sur un support, lequel cône est revêtu d'un partenaire de liaison, une barrette comprenant 10 puits (donc 10 récipients) dont un puits échantillon, un puits pour la stimulation, un puits pour la lecture du signal représentatif de la détection de la molécule effectrice immune et des puits pouvant contenir les différents constituants, par exemple ceux nécessaires à la stimulation et à l'immunoessai mis en œuvre pour la détection de la culture cellulaire immune. Cet instrument possède également un système de régulation de la température des puits de la barrette. L'instrument VIDAS^{®} 3 possède également un bras muni d'un embout.

Comme indiqué précédemment, le procédé de détection de la réponse cellulaire immune de l'invention peut être divisé en deux phases, dont la premier phase constitue un autre objet de l'invention, qui est un procédé automatisé de stimulation d'au moins une cellule susceptible de sécréter une molécule effectrice immune pour la sécrétion de ladite molécule, ladite au moins une cellule étant issue d'un échantillon biologique d'origine animale, caractérisé en ce qu'il comprend ou consiste en les étapes consistant à :
(a) aspirer une quantité définie de l'échantillon biologique tel que prélevé par un dispositif automatisé d'aspiration/refoulement,
(b) refouler cette quantité définie, à l'aide dudit dispositif automatisé d'aspiration/refoulement, dans un récipient R, ledit récipient R contenant au préalable au moins un stimulant de sécrétion de ladite molécule par ladite au moins une cellule ou bien ledit récipient R ne contenant pas un tel au moins un stimulant,
(c) lorsque le récipient ne contient pas au préalable au moins un stimulant de sécrétion, introduire dans ledit récipient R à l'aide dudit dispositif automatisé d'aspiration/refoulement, ledit au moins un stimulant de sécrétion contenu dans un récipient E, et
(d) laisser incuber la quantité définie d'échantillon et ledit au moins un stimulant, formant un mélange, pour la sécrétion de ladite molécule par ladite au moins une cellule (i) dans ledit récipient R ou (ii) dans un autre récipient S après aspiration et refoulement dudit mélange à l'aide dudit dispositif automatisé d'aspiration/refoulement.

Les caractéristiques liées à l'échantillon biologique, aux cellules à cultiver, à la molécule effectrice immune, également appelée molécule d'intérêt ou encore analyte, aux maladies d'intérêt, aux conditions de stimulation/incubation, avec ou sans stimulant(s) spécifique(s) de pathogène, aux dispositifs d'automatisation utilisés, aux méthodes de détection de la réponse immune, etc., telles que décrites précédemment dans le cadre du procédé de détection de l'invention s'appliquent également pour le procédé de stimulation.

La molécule ainsi obtenue par stimulation automatisée et sa détection sont particulièrement utiles dans le domaine médical. Aussi l'invention concerne également l'utilisation du procédé de détection d'une réponse cellulaire immune ou du procédé automatique de stimulation tels que décrits précédemment pour le diagnostic et le suivi de pathologies, pour les études de toxicité de médicaments et vaccins et pour déterminer le statut immunitaire d'un patient.

Les pathologies concernées ont été décrites précédemment. Parallèlement, il est important que les médicaments et vaccins, futurs ou non, ne soient pas toxiques et les procédés de l'invention peuvent être utilisés en ce sens. Enfin comme le statut immunitaire d'un patient est évalué via la capacité des cellules impliquées dans la réponse immunitaire à sécréter des molécules effectrices immunes, le procédé de l'invention est particulièrement bien adapté dans ce but.

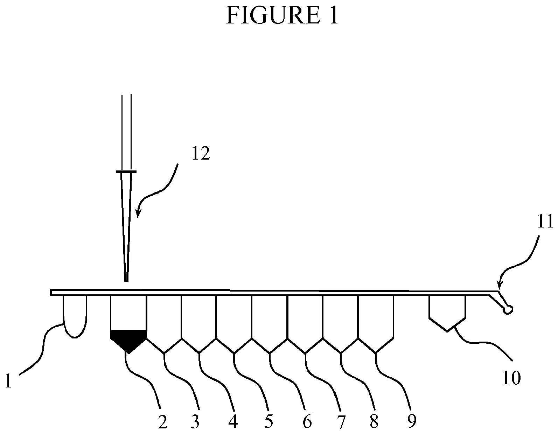

L'invention sera mieux comprise à l'aide des exemples suivants qui sont donnés à titre illustratif et non limitatif, ainsi qu'à l'aide de la Figure 1 qui est une représentation schématique du cône et de la barrette utilisés avec l'instrument VIDAS^{®}, le cône appartenant au dispositif d'aspiration/refoulement pour aspirer et refouler la quantité définie d'échantillon biologique dans un puits de stimulation.

### EXEMPLES

### Exemple 1 : Automatisation de l'étape de stimulation, prélèvement de sang total, stimulation avec un stimulant non spécifique, dosage de l'interféron gamma

### 1.1. Mise en œuvre de l'étape de sécrétion

Les échantillons de sang utilisés dans cet exemple ont été obtenus auprès des Etablissements Français du Sang (EFS) et collectés chez des donneurs sains. Cinq échantillons de sang ont été prélevés stérilement dans des tubes Vacutainer^{®} (Becton-Dickinson) contenant de l'héparinate de lithium ou référence équivalente chez un autre fournisseur.

### Ces échantillons ont été stimulés selon deux conditions (1 et 2)

*Condition 1 : Stimulation en tube, selon l'art antérieur* - *conditions stériles de stimulation.* On utilise pour cette condition des tubes secs stériles (Cat. No. 367614, Becton-Dickinson). En condition stérile, sous une hotte à flux laminaire, chaque échantillon sanguin est stimulé de 3 façons différentes:
Essai 1 : Tube PBS (contrôle négatif) : 400 µL de tampon PBS sont mélangés avec 400 µL de sang total.

Essai 2 : Tube phorbol 12-myristate 13-acétate (PMA) - phytohemagglutinin (PHA) : 400 µL de stimulants PMA (2,30 ng/ml) et PHA (0,46 µg/ml) sont mélangés avec 400 µL de sang total.

Essai 3 : Tube phorbol 12-myristate 13-acétate (PMA) - phytohemagglutinin (PHA) : 400 µL de stimulants PMA (25 ng/ml) et PHA (5 µg/ml) sont mélangés avec 400 µL de sang total.

Les tubes ont été rebouchés sous hotte puis incubés verticalement à 37°C pendant 17h30 heures.

*Condition 2 : Stimulation automatisée en cartouche VIDAS*^{®} - *conditions non stériles de stimulation.* La barrette de l'automate d'immunoanalyse VIDAS^{®} est décrite sur la figure 1. Celle-ci (11) est composée de 10 puits (1 à 10) recouverts partiellement d'une feuille d'aluminium scellée étiquetée (non montrée). Le dernier puits (10) est une cuvette optique dans laquelle la fluorescence est mesurée et contient le substrat enzymatique. Les différents réactifs nécessaires à la stimulation et/ou l'analyse comme le stimulant (ici puits PBS ou puits PMA-PHA), les solutions de lavage ou l'anticorps de révélation, sont contenus dans les différents autres puits (1 à 9).

Les barrettes ainsi préparées ont été insérées dans l'automate VIDAS^{®} 3 ainsi que les tubes de sang total prélevés chez les donneurs. Avant de charger les tubes dans l'automate, leur bouchon est retiré. A partir de cet instant, les échantillons de sang total ne sont plus traités en condition stérile. L'instrument VIDAS^{®} 3 a été programmé afin d'effectuer automatiquement les étapes suivantes :
1) Aspirer par le dispositif d'aspiration/refoulement un volume précis de sang total (400 µL) à partir du tube de prélèvement primaire
2) Percer la feuille d'aluminium qui couvre le puits de stimulation à l'aide du dispositif d'aspiration/refoulement,
3) Refouler le sang total dans le puits de stimulation
4) Incuber la cartouche à 37°C pendant 17h30.

A la suite de cette étape de stimulation, le VIDAS^{®} 3 peut automatiquement enchainer avec l'étape de détection de l'IFNγ sécrété, selon le mode opératoire décrit ci-dessous dans le paragraphe 1.2.

Les conditions de stimulation en tube stérile (tube) ou dans la barrette du VIDAS^{®} (puits) sont résumées dans le Tableau 1 ci-après.

**Tableau 1 : conditions de stimulation en tube stérile ou en puits d'automate**

| Conditions | Contenant stimulation | Stimulant de sécrétion |
|---|---|---|
| PBS | Tube | PBS (Contrôle négatif) |
| PMA-PHA concentration faible | Tube | PMA 2,30 ng/ml + PHA 0,46 µg/ml en PBS |
| PMA-PHA concentration forte | Tube | PMA 25 ng/ml + PHA 5 µg/ml en PBS |
| PBS | Puits | PBS (Contrôle négatif) |
| PMA-PHA concentration faible | Puits | PMA 2,30 ng/ml + PHA 0,46 µg/ml en PBS |
| PMA-PHA concentration forte | Puits | PMA 25 ng/ml + PHA 5 µg/ml en PBS |

| | | |
|---|---|---|
| PMA : phorbol 12-myristate 13-acétate PHA : phytohemagglutinin | | |

### 1.2. Etape de détection de l'IFNγ : mode opératoire de l'immunoessai automatisé

### Principe de détection

La détection de l'IFNγ dans les échantillons biologiques est réalisée par immunoessai sandwich en une étape en utilisant l'automate d'immunoanalyse VIDAS^{®} (bioMérieux). Le cône à usage unique (représenté en 12 sur la Figure 1) sert à la fois de phase solide pour la réaction et de système de pipetage. Il est utilisé en combinaison avec la barrette (11), comme décrit dans le point 1.1 ci-dessus. Toutes les étapes du test sont ainsi réalisées automatiquement par l'instrument. Elles sont constituées d'une succession de cycles d'aspiration/refoulement du milieu réactionnel.

*Sensibilisation et passivation des cônes.* Les cônes ont été sensibilisés avec 270 µL d'une solution d'un premier anticorps monoclonal anti-IFNy (4G1E12, bioMérieux, France) à 5 µg/ml dans un tampon carbonate. Après environ 6-8h d'incubation à +18/25°C avec la solution de sensibilisation, les cônes ont été vidés. Ensuite, 300 µL de tampon phosphate contenant 10 g/L d'albumine bovine sont ajoutés pour la passivation des cônes à +18/25°C pendant environ 18-24h. Les cônes sont ensuite vidés, séchés, puis conservés à +4°C jusqu'à utilisation, à l'abri de l'humidité.

*Préparation des solutions d'anticorps conjugués.* La solution de conjugué (partenaire de détection) contient un second anticorps monoclonal anti-IFNy couplé à la phosphatase alcaline (2A4B11, bioMérieux). Les anticorps conjugués ont été dilués à environ 500 ng/ml dans un tampon Phosphate/BSA.

*Immunoessai.* Dès que le cône VIDAS^{®} est en contact avec l'échantillon, la réaction immunologique commence car les anticorps de capture sont immobilisés sur ce cône. L'automate mélange l'échantillon à tester (100 µL - dans le cas présent le sang après stimulation) avec 250 µL de la solution de conjugué. L'incubation dure environ 10 minutes à 37°C et permet la liaison spécifique de l'IFNγ à l'anticorps adsorbé sur le cône d'une part (anticorps de capture) et à l'anticorps conjugué (anticorps de détection) d'autre part. Ensuite, les composants non liés sont éliminés par 3 lavages avec un tampon Tris NaCl. Lors de l'étape finale de révélation, le substrat 4-méthylombelliferyl phosphate est aspiré puis refoulé dans le cône ; l'enzyme des anticorps conjugués catalyse la réaction d'hydrolyse de ce substrat en 4-méthylombelliferone dont la fluorescence émise est mesurée à 450 nm. La valeur du signal de fluorescence (RFV : relative fluorescence value) est proportionnelle à la concentration de l'antigène présent dans l'échantillon.

Les résultats des dosages sont présentés dans le tableau 2 :

**Tableau 2 : mesure de l'IFNγ par VIDAS**

| | | Mesure IFNγ par VIDAS Signal en RFV | | | | |
|---|---|---|---|---|---|---|
| Code échantillon | **Contenant de stimulation** | PBS | PMA/PHA concentration forte | PMA/PHA concentration faible | **Ratio PMA-PHA concentration forte / PBS** | **Ratio PMA-PHA concentration faible / PBS** |
| 0312274 | **Tube** | 23 | 11447 | 229 | **498** | **10** |
| | **Puits** | 11 | 11516 | 72 | **1047** | **7** |
| 0312370 | **Tube** | 15 | 11319 | 111 | **755** | **7** |
| | **Puits** | 8 | 10928 | 48 | **1366** | **6** |
| 0312397 | **Tube** | 26 | 11216 | 767 | **431** | **30** |
| | **Puits** | 16 | 13588 | 543 | **43,6** | **34** |
| 0312303 | **Tube** | 16 | 11306 | 610 | **707** | **38** |
| | **Puits** | 7 | 8905 | 172 | **1272** | **25** |

Une très forte sécrétion d'INFy (signaux > 8000 RFV) est observée lorsque les cellules sont stimulées en utilisant une concentration forte de PHA dans le mélange PMA/PHA alors que la sécrétion est modérée (signaux < 800 RFV) si la concentration de PHA est faible dans le mélange PMA/PHA. Ce phénomène est observé tant pour la stimulation réalisée dans le tube que pour celle effectuée dans le puits.

La sécrétion non spécifique en l'absence de stimulant (essai PBS) est très faible en tube (signaux < 30 RFV) et quasiment inexistante dans le puits (signaux < 20 RFV).

Le niveau de sécrétion d'INFy obtenu après stimulation est comparé à celui mesuré en l'absence de stimulant en calculant un rapport entre les 2 mesures. Si le rapport est d'au moins 3, on conclut positivement.

Lorsque la stimulation est réalisée dans le puits, les rapports varient de 6 à 1366 alors que, lorsque la stimulation est réalisée dans le tube, les rapports varient de 7 à 755. On peut donc conclure que la stimulation entrainant la sécrétion d'INFy est équivalente ou meilleure dans le puits que dans le tube.

### Exemple 2 : Automatisation de l'étape de stimulation, prélèvement de sang total, stimulation avec un stimulant spécifique (antigènes Mycobacterium tuberculosis), dosage de l'interféron gamma

Les échantillons de sang utilisés dans cet exemple ont été obtenus auprès de Biotech Bank (Angers, France) et collectés soit chez des personnes ayant eu une tuberculose soit dans leur entourage proche. Quatre échantillons de sang ont été prélevés stérilement dans des tubes Vacutainer^{®} (Becton-Dickinson) contenant de l'héparinate de lithium ou référence équivalente chez un autre fournisseur.

Chaque échantillon de sang a été stimulé selon 3 protocoles différents détaillés dans le Tableau 3 ci-après, en utilisant comme agent de stimulation le contenu des tubes Quantiferon^{®} TB (Cat. No. T0590-0301, Qiagen). Les tubes Quantiferon^{®} TB sont des tubes de prélèvement de sang sous vide, particuliers, qui permettent le recueil du sang total dans des conditions parfaitement stériles. Ils sont au nombre de 3 :
Le tube AG, pour antigène, contient des peptides issus des protéines ESAT-6, CFP-10 et TB7.7 de *Mycobacterium tuberculosis* ; il s'agit du tube pour la stimulation spécifique. S'il y a sécrétion d'IFNγ dans ce tube, c'est que l'échantillon contient des cellules T qui sont capables de réagir spécifiquement contre les peptides de *M. tuberculosis.*

Le tube NIL correspond au contrôle négatif et le tube MIT, qui contient un mitogène, correspond au contrôle positif.

Chaque échantillon a été stimulé par le contenu des 2 tubes AG et NIL Quantiferon et selon 2 protocoles, ce qui correspond à un total de 4 conditions par échantillon.

En utilisation de routine, selon les instructions du fabricant, 0,8 à 1,2 mL de sang total sont directement prélevés sur les tubes Quantiferon^{®} TB de manière stérile et la phase de stimulation se fait de façon stérile dans les tubes contenant au préalable les stimulants de sécrétion. Puis, les échantillons ainsi stimulés sont centrifugés et la détection de l'interféron gamma est mise en œuvre en microplaque dans le plasma ainsi obtenu. Cependant afin de pouvoir effectuer la comparaison avec la stimulation automatisée, nous avons légèrement modifié ce mode opératoire, comme indiqué dans le Tableau 3 :

**Tableau 3 : conditions de stimulation et de détection de l'interféron gamma**

| | |
|---|---|
| Condition de référence CENTRIFUGATION | 1. Sous une hotte à flux laminaire, en condition stérile : ajout de 400 µL de PBS dans chaque tube Quantiferon, puis ajout de 400 µL de sang |
| | 2. 10 retournements pour mélanger |
| | 3. Incubation en position verticale à 37°C pendant 17h30 |
| | 4. Centrifugation pendant 15 min à 1800g |
| | 5. Détection IFNγ dans le plasma par VIDAS |
| Condition automatisée, selon l'invention | 1. Ajout de 400 µL de PBS dans chaque tube Quantiferon |
| | 2. 10 retournements pour mélanger |
| | 3. Récupération de 400 µL de stimulant pour transfert dans le puits de stimulation de la cartouche VIDAS |
| | 4. Lancement du programme automatisé de stimulation (non stérile) + incubation + dosage sur VIDAS : |
| | o Aspiration automatique de 2x200 µL de sang total à partir du tube primaire |
| | o Refoulement des 400 µL dans le puits de stimulation |
| | o Incubation à 37°C pendant 17h30 |
| | o Détection IFNγ dans l'échantillon ainsi stimulé par VIDAS |

La détection de l'IFNγ dans les échantillons de sang après stimulation a été réalisée selon le mode opératoire détaillé dans le paragraphe 1.2 de l'Exemple 1.

Les résultats (signal RFV mesuré par le VIDAS^{®}) selon les conditions expérimentales) sont donnés dans le tableau 4 ci-après :

**Tableau 4 : mesure de l'IFNγ par VIDAS**

| | | Mesure INFγ par VIDAS Signal en RFV | | | |
|---|---|---|---|---|---|
| Code patient | **Protocole de stimulation** | NIL | ANTIGEN (AG) | **Ratio AG/NIL** | **Interprétation** |
| BK-37 | **Référence** | 104 | 118 | **1,1** | **Neg** |
| | **Automatisé** | 121 | 204 | **1,7** | **Neg** |
| BK-38 | **Référence** | 14 | 15 | **1,1** | **Neg** |
| | **Automatisé** | 11 | 14 | **1,3** | **Neg** |
| BK-39 | **Référence** | 53 | 482 | **9,1** | **Pos** |
| | **Automatisé** | 31 | 1351 | **43,6** | **Pos** |
| BK-40 | **Référence** | 67 | 354 | **5,3** | **Pos** |
| | **Automatisé** | 64 | 268 | **4,2** | **Pos** |
| BK-33 | **Référence** | 42 | 118 | **2.8** | **Neg** |
| | **Automatisé** | 45 | 48 | **1.1** | **Neg** |
| BK-34 | **Référence** | 98 | 98 | **1.0** | **Neg** |
| | **Automatisé** | 113 | 142 | **1.3** | **Neg** |
| BK-20 | **Référence** | 179 | 1083 | **6.1** | **Pos** |
| | **Automatisé** | 114 | 1954 | **17.1** | **Pos** |

*Résultats* : Le protocole de stimulation et détection de la réponse cellulaire immune automatisé, sans centrifugation et en condition non stérile, permet d'obtenir des signaux (11-1954 RFV) équivalents ou supérieurs à ceux obtenus avec le protocole de référence en tube stérile (14-1083 RFV). Le ratio AG/NIL varie de 1,1 à 43,6 pour la condition automatisée et de 1,1 à 17,1 pour la condition référence, ce qui traduit une stimulation spécifique équivalente ou supérieure en condition automatisée.

Les échantillons BK-37, BK-38, BK-33 et BK-34 ont des ratios AG/NIL qui varient entre 1,0 et 2,8 dans les 2 conditions testées. Le niveau de sécrétion l'IFNγ détecté est basal (ratio < 3) et n'est pas spécifique des antigènes de *M. tuberculosis.* Par conséquent la condition automatisée et la condition référence permettent de conclure que ces patients n'ont pas été infectés par *M. tuberculosis* : interprétation négative (Neg).

L'échantillon BK-40 présente des ratios AG/NIL entre 4,2 et 5,3 selon le protocole de stimulation, très concordants. Dans cet échantillon, une sécrétion d'IFNy (ratio > 3) déclenchée spécifiquement par les antigènes de *M. tuberculosis* est observée. Nous pouvons en conclure que le patient BK-40 a déjà été infecté par *M. tuberculosis* : interprétation positive (Pos). La même interprétation est obtenue pour les échantillons BK-39 et BK-20 pour lesquels les plus hauts niveaux de sécrétion spécifique d'IFNy sont mis en évidence. Pour ces patients, les ratios AG/NIL ne sont pas équivalents entre les 2 conditions : des ratios de 43,6 et 17,1 sont respectivement observés pour la condition automatisée contre 9,1 et 6,1 pour la condition référence, ce qui montre une sensibilité analytique améliorée pour la condition automatisée, sans étape de centrifugation et en condition non stérile avec une quantité de sang calibrée, comparée à la condition de référence (conditions stériles avec quantité de sang qui varie).

### Exemple 3 : Automatisation de l'étape de stimulation, prélèvement de sang total, stimulation non spécifique PMA + PHA, dosage du TNF alpha

### 3.1. Mise en œuvre de l'étape de sécrétion

Les échantillons de sang utilisés dans cet exemple ont été obtenus auprès des Etablissements Français du Sang (EFS) et collectés chez des donneurs sains. 2 échantillons de sang ont été prélevés stérilement dans des tubes Vacutainer^{®} (Becton-Dickinson) contenant de l'héparinate de lithium ou référence équivalente chez un autre fournisseur et stimulés soit en tube (condition stérile référence), soit en puits VIDAS^{®} (selon l'invention) selon le mode opératoire décrit dans le paragraphe 1.1.

Les conditions de stimulation en tube stérile (tube) ou dans la barrette du VIDAS^{®} (puits) sont résumées dans le Tableau 5 ci-après :

**Tableau 5 : conditions de stimulation en tube stérile ou en puits d'automate**

| Conditions | Contenant stimulation | Stimulant de sécrétion |
|---|---|---|
| PBS | Tube | PBS (Contrôle négatif) |
| PMA-PHA Concentration forte | Tube | PMA 25 ng/ml + PHA 5 µg/ml en PBS |
| PBS | Puits | PBS (Contrôle négatif) |
| PMA-PHA Concentration forte | Puits | PMA 25 ng/ml + PHA 5 µg/ml en PBS |

A l'issue de l'étape de stimulation, l'échantillon est prélevé afin de mesurer la concentration de TNFα avec le kit Quantikine^{®} HS ELISA human TNFα immunoassay de R&D systems (Cat No. HSTA00D) en appliquant le mode opératoire préconisé par le fabricant (notice version 12/13).

### 3.2. Résultats du dosage du TNF alpha

Les résultats expérimentaux obtenus sont présentés dans le Tableau 6 ci-dessous :

**Tableau 6 : mesure du TNFα par ELISA**

| | | Mesure TNFα par ELISA en pg/ml | | |
|---|---|---|---|---|
| Code échantillon | **Contenant stimulation** | PBS | PMA/PHA Concentration forte | **Ratio PMA-PHA Concentration forte/PBS** |
| 59171003916 | **Tube** | 2.15 | > 32 | > 14,9 |
| | **Puits** | 2.95 | > 32 | > 10,8 |
| 5917100373- | **Tube** | 1.33 | > 32 | > 24, 1 |
| | **Puits** | 0.97 | > 32 | > 33,0 |

Comme le montre le tableau 6, une très forte sécrétion de TNFα (concentration supérieure à 32 pg/mL) est observée lorsque les cellules sont stimulées en utilisant une concentration forte de PHA dans le mélange PMA/PHA. Ce phénomène est observé tant pour la stimulation réalisée dans le tube que pour celle effectuée dans le puits.

La sécrétion non spécifique en l'absence de stimulant (essai PBS) est très faible tant en tube que dans le puits (concentration < 3 pg/mL).

Lorsque la stimulation est réalisée dans le puits, les rapports sont de 10,8 et 33, comparables à ceux calculés lorsque la stimulation est réalisée dans le tube, respectivement 14,9 et 24,1. On peut donc conclure que la sécrétion de TNFα a été stimulée par le mélange PMA-PHA de façon équivalente dans le tube et le puits.

### Exemple 4 : Automatisation de l'étape de stimulation, prélèvement de sang total, stimulation non spécifique LPS, dosage du TNF alpha

### 4.1. Mise en œuvre de l'étape de sécrétion

Les échantillons de sang utilisés dans cet exemple ont été obtenus auprès des Etablissements Français du Sang (EFS) et collectés chez des donneurs sains. 2 échantillons de sang ont été prélevés stérilement dans des tubes Vacutainer^{®} (Becton-Dickinson) contenant de l'héparinate de lithium ou référence équivalente chez un autre fournisseur.

Les échantillons ont été stimulés soit en tube (condition stérile REF), soit en puits VIDAS^{®} (selon l'invention), selon le mode opératoire décrit dans le paragraphe 1.1 de l'exemple 1, à la différence près que le mélange PMA+PHA a été remplacé par un mélange à parts égales de lipopolysaccharides de *E. coli* (LPS, Sigma, Cat. No. L3012, L2637 et L3137). Les conditions de stimulation sont récapitulées dans le tableau 7 :

**Tableau 7 : conditions de stimulation en tube stérile ou en puits d'automate**

| Conditions | Contenant stimulation | Stimulant de sécrétion |
|---|---|---|
| PBS | Tube | PBS (Contrôle négatif) |
| LPS | Tube | LPS 50 ng/ml |
| PBS | Puits | PBS (Contrôle négatif) |
| LPS | Puits | LPS 50 ng/ml |

A l'issue de l'étape de stimulation, l'échantillon est prélevé afin de mesurer la concentration de TNF alpha avec le kit Quantikine^{®} HS ELISA human TNF alpha immunoassay de R&D systems (Cat No. HSTA00D) en appliquant le mode opératoire préconisé par le fabricant (notice version 12/13).

### 4.2. Résultats du dosage du TNF alpha

Les résultats expérimentaux obtenus sont présentés dans le Tableau 8 :

**Tableau 8 : mesure du TNFα par ELISA**

| | | Mesure TNFα par ELISA en pg/ml | | |
|---|---|---|---|---|
| Code échantillon | **Contenant stimulation** | PBS | PMA/PHA Concentration forte | **Ratio PMA-PHA Concentration forte/PBS** |
| 59171003916 | **Tube** | 2.15 | > 32 | > 14,9 |
| | **Puits** | 2.95 | > 32 | > 10,8 |
| 5917100373- | **Tube** | 1.33 | > 32 | > 24, 1 |
| | **Puits** | 0.97 | > 32 | > 33,0 |

Comme le montre le tableau 8, une très forte sécrétion de TNFα (concentration > 32 pg/mL) est observée lorsque les cellules sont stimulées en utilisant du LPS. Ce phénomène est observé tant pour la stimulation réalisée dans le tube que pour celle effectuée dans le puits.

La sécrétion non spécifique en l'absence de stimulant (essai PBS) est très faible tant en tube que dans le puits (concentration < 3 pg/mL).

Lorsque la stimulation est réalisée dans le puits, les rapports sont de 10,8 et 33 comparables à ceux calculés lorsque la stimulation est réalisée dans le tube, respectivement 14,9 et 24,1. On peut donc conclure que la sécrétion de TNFα a été stimulée par le LPS de façon équivalente dans le tube et le puits.

### Exemple 5 : Automatisation de l'étape de stimulation, prélèvement de sang total, stimulation avec un stimulant spécifique (antigènes Cytomégalovirus ), dosage de l'interféron gamma

Les échantillons de sang utilisés dans cet exemple ont été obtenus auprès de l'Etablissement Français du Sang (EFS Rhône-Alpes, France). Treize échantillons de sang ont été prélevés stérilement dans des tubes Vacutainer^{®} (Becton-Dickinson) contenant de l'héparinate de lithium ou référence équivalente chez un autre fournisseur.

Chaque échantillon de sang a été stimulé selon le protocole « Condition automatisée, selon l'invention » décrite dans le Tableau 3 de l'exemple 2 en utilisant comme agent de stimulation le contenu des tubes Quantiferon^{®} CMV (Cat. No.0192-0301, Qiagen). Les tubes Quantiferon^{®} CMV sont des tubes de prélèvement de sang sous vide, particuliers, qui permettent le recueil du sang total dans des conditions parfaitement stériles. Ils sont au nombre de 3 :
Le tube AG, pour 'antigène, contient des peptides du Cytomégalovirus; il s'agit du tube pour la stimulation spécifique. S'il y a sécrétion d'IFNy lors de cette stimulation, c'est que l'échantillon de sang contient des cellules T qui sont capables de réagir spécifiquement contre les peptides du Cytomégalovirus.

Le tube NIL correspond au contrôle négatif et le tube MIT, qui contient un mitogène, correspond au contrôle positif.

Chaque échantillon a été stimulé par le contenu des tubes AG et NIL Quantiferon.

La détection de l'IFNγ dans les échantillons de sang après stimulation a été réalisée selon le mode opératoire détaillé dans le paragraphe 1.2 de l'Exemple 1.

Les résultats (signal RFV mesuré par le VIDAS^{®}) selon les conditions expérimentales) sont donnés dans le tableau 9 ci-après.

**Tableau 9 : mesure de l'IFN y par VIDAS^{®}**

| | Mesure INF γ par VIDAS^{®} Signal en RFV | | | |
|---|---|---|---|---|
| Code échantillon | NIL | ANTIGEN (AG) | **Ratio AG/NIL** | **Interprétation** |
| 59180446129 | 87 | 90 | **1,0** | **Neg** |
| 59180447383 | 24 | 26 | **1,1** | **Neg** |
| 59180447498 | 11 | 15 | **1,4** | **Neg** |
| 59180447500 | 47 | 41 | **0,9** | **Neg** |
| 59180404025 | 12 | 2505 | **209** | **Pos** |
| 59180404005 | 25 | 26 | **1,0** | **Neg** |
| 59180404324 | 3 | 2325 | **775** | **Pos** |
| 59180404113 | 20 | 27 | **1,4** | **Neg** |
| 59180404076 | 23 | 7463 | **324** | **Pos** |
| 59174894224 | 9 | 12053 | **1339** | **Pos** |
| 59174894208 | 14 | 54 | **3,9** | **Neg** |
| 59174890039 | 98 | 1686 | **17,2** | **Pos** |
| 59174890020 | 26 | 29 | **1,1** | **Neg** |

*Résultats* : Le protocole de stimulation et détection de la réponse cellulaire immune automatisé, sans centrifugation et en condition non stérile, permet d'obtenir des signaux de 3 à 12053 RFV et des ratios AG/NIL variant de 0,9 à 1339.

Les échantillons 59180446129, 59180447383, 59180447498, 59180447500, 5918040405-, 59180404113, 59174894208 et 59174890020 ont des ratios AG/NIL qui varient entre 0,9 à 3,9. Le niveau de sécrétion l'IFNγ détecté est basal (ratio < 5) et n'est pas induit par les peptides du Cytomégalovirus. Par conséquent la stimulation en condition automatisée permet de conclure que ces patients n'ont pas été infectés par le Cytomégalovirus : interprétation négative (Neg).

Les échantillons 59180404025, 59180404324, 59180404076, 59174894224 et 59174890039 présentent des ratios AG/NIL entre 17,2 et 1339. Dans ces échantillons, une sécrétion d'IFNy (ratio > 5) déclenchée spécifiquement par les peptides du Cytomégalovirus est observée. Par conséquent la stimulation en condition automatisée permet de conclure que ces patients ont été infectés par Cytomégalovirus : interprétation positive (Pos).

### Exemple 6 : Automatisation de l'étape de stimulation, prélèvement de sang total, stimulation avec un stimulant spécifique (antigènes du virus d'Epstein-Barr)

Les échantillons de sang utilisés dans cet exemple ont été obtenus auprès l'Etablissement Français du Sang (EFS, Rhône-Alpes, France). Dix échantillons de sang ont été prélevés stérilement dans des tubes Vacutainer^{®} (Becton-Dickinson) contenant de l'héparinate de lithium ou référence équivalente chez un autre fournisseur.

Chaque échantillon de sang a été stimulé selon le protocole « Condition automatisée, selon l'invention » détaillé dans le Tableau 3 de l'exemple 2, en utilisant comme agent de stimulation des peptides du virus d'Epstein-Barr (Pep Tivator^{®}). Pour la condition NIL, l'échantillon a été mis en contact uniquement avec du PBS.

La détection de l'IFNγ dans les échantillons de sang après stimulation a été réalisée selon le mode opératoire détaillé dans le paragraphe 1.2 de l'Exemple 1.

Les résultats (signal RFV mesuré par le VIDAS^{®} selon les conditions expérimentales) sont donnés dans le tableau 10 ci-après.

**Tableau 10 : mesure de l'IFNγ par VIDAS^{®}**

| | Mesure INF g par VIDAS^{®} Signal en RFV | | | |
|---|---|---|---|---|
| Code échantillon | NIL | ANTIGEN (AG) | **Ratio AG/NIL** | **Interprétation** |
| 59174878275 | 19 | 727 | **38,3** | **Pos** |
| 59174878101 | 49 | 1382 | **28,2** | **Pos** |
| 59174878136 | 37 | 4838 | **131** | **Pos** |
| 59174878208 | 47 | 21 | **0,4** | **Neg** |
| 59174878179 | 7 | 832 | **119** | **Pos** |
| 59180477726 | 15 | 268 | **17,9** | **Pos** |
| 59180477734 | 11 | 22 | **2,0** | **Neg** |
| 59180477718 | 32 | 1263 | **39,5** | **Pos** |
| 59180477697 | 83 | 79 | **1,0** | **Neg** |
| 5918047770 | 41 | 1163 | **28,4** | **Pos** |

*Résultats* : Le protocole de stimulation et détection de la réponse cellulaire immune automatisé, sans centrifugation et en condition non stérile, permet d'obtenir des signaux de 7 à 4838 RFV et des ratios AG/NIL variant de 0,4 à 131.

Les échantillons 59174878208, 59180477734 et 59180477697 ont des ratios AG/NIL qui varient entre 0,4 et 2,0. Ces ratios sont faibles (< 4), ce qui signifie que la sécrétion d'IFNy n'est pas liée aux peptides du virus EBV. Par conséquent la stimulation en condition automatisée permet de conclure que ces patients n'ont pas été infectés par le virus d'Epstein-Barr : interprétation négative (Neg).

Les échantillons 59174878275, 59174878101, 59174878136, 59174878179, 59180477726, 59180477718 et 5918047770- présentent des ratios AG/NIL entre 17,9 et 131. Ces ratio sont forts (> 4), ce qui signifie que la sécrétion d'IFNy est liée spécifiquement aux peptides du virus d'Epstein-Barr. Par conséquent la stimulation en condition automatisée permet de conclure que ces patients ont été infectés par le virus d'Epstein-Barr : interprétation positive (Pos).

### Exemple 7 : Automatisation de l'étape de stimulation, prélèvement de sang total, stimulation non spécifique LPS, dosage par biologie moléculaire

Les échantillons de sang utilisés dans cet exemple ont été obtenus auprès l'Etablissement Français du Sang (EFS, Rhône-Alpes, France). Cinq échantillons de sang ont été prélevés stérilement dans des tubes Vacutainer^{®} (Becton-Dickinson) contenant de l'héparinate de lithium ou référence équivalente chez un autre fournisseur.

Chaque échantillon de sang a été stimulé selon le protocole « Condition automatisée, selon l'invention » détaillé dans le Tableau 3 de l'exemple 2, en utilisant comme agent de stimulation du LPS. Pour la condition NIL, l'échantillon a été mis en contact uniquement avec du PBS.

La détection de la réponse cellulaire immune par quantification des ARNm des gènes suivants : gène de l'IFNy, gène de l'IL1β et gène de ZAP70 (kit internes bioMérieux), dans les échantillons de sang après stimulation, a été réalisée comme suit : les ARNm ont été extraits après lyse de 100µL du mélange réactionnel en utilisant une méthode automatisée (technologie BOOM). Les ARNm ont ensuite été amplifiés par RT-nested PCR à 2 étapes (1^{ère} PCR de 26 cycles, suivies d'une 2^{ème} PCR de 35 cycles). La détection proprement dite a été réalisée en utilisant un intercalant et les mesures en temps réel ont été acquises pour construire une courbe d'amplification et obtenir un point Cp (« crossing point ») pour chaque cible et pour 3 gènes de référence (DECR1, PPIB & FPGS). Le signal, correspondant au niveau d'expression de l'ARNm, a été normalisé en soustrayant le Cp cible par une moyenne géométrique des 3 gènes de référence.

Les résultats (valeurs de Cp normalisées ± déviation standard) sont donnés dans le tableau 11 ci-après. Plus la valeur normalisée Cp est faible, plus le niveau d'expression est élevé.

**Tableau 11**

| | Valeurs de Cp normalisées ± SD | | P-value |
|---|---|---|---|
| | NIL | LPS | |
| IFN-y | 5,33 ± 0,88 | 3,12 ± 1,19 | 0,0159 |
| IL1B | -4,37 ± 4,04 | -7,90 ± 0,69 | 0,1508 |
| ZAP70 | -1,75 ± 0,61 | -2,14 ± 0,86 | 0,5476 |

Les résultats du tableau 11 ci-dessous montrent qu'il est possible de détecter une réponse cellulaire immune par test de biologie moléculaire après stimulation automatisée selon l'invention.

### Références Bibliographiques

- Almeida CA. M. et al, 2009, Journal of Immunological Methods, 344 : 1-5
- Boersma YL et Plütckthun A, 2011, Curr. Opin. Biotechnol, 22 : 849-857
- Bustin SA, 2002, Journal of molecular endocrinology, 29 : 23-39
- Coligan J.E., 1994, Current Protocols in Immunology, Volume I, Supplément 10, Unit 6.19 : 1-8
- Cox J.H. et al., 2006, Methods, 38 : 274-282
- Ellington AD et Szostak JW., 1990, Nature, 346 : 818-822
- Gaur RL, et al., 2013, Journal of Clinical Microbiology, 51(11) : 3521-3526
- Giulietti A et al., 2001, Methods, 25 : 386-401
- Handbook of Elispot, 2005, Methods and Protocols, Humana Press Totowa, New Jersey, 1-321
- Keller GH and Manak MM, 1993. DNA Probes, p. 137-196. Stockton Press, Stockton Press
- Kricka LJ, 1999, Nucleic acid détection technologies -- labels, strategies, and formats, Clin. Chem. 45, 453-458
- Lalvani, A., 2007, Chest, 131 : 1899-1906
- Neubauer J.C. et al., 2017, Cytotechnology, 69(1) : 57-73
- Pal M. et al., 2014, Clin. Microbiol. Rev., 27(1) : 3-20
- Rassasie M.J. et al., 1992, Steroids, 57: 112
- Revue générale, 2003, Ann Biol Clin, 61 : 635-44
- Stabler T.V., et al., 1991, Clin. Chem., 37(11): 1987
- Uemori T, 2007, J Biochem. Aug, 142(2): 283-92
- Vincent et al., 2004, Embo reports, 5(8) : 795-800
- Wood P.R., 1990, Research in Veterinary Science, 49 : 46-49

## Revendications

1. Procédé de détection d'une réponse cellulaire immune dans un échantillon biologique d'origine animale, susceptible de contenir au moins une cellule sécrétrice d'au moins une molécule effectrice immune, comprenant les étapes consistant à :
(a) aspirer une quantité définie de l'échantillon biologique qui a été préalablement prélevé, par un dispositif automatisé d'aspiration/refoulement,
(b) refouler cette quantité définie, à l'aide dudit dispositif automatisé d'aspiration/refoulement, dans un récipient R, ledit récipient R contenant au préalable au moins un stimulant de sécrétion de ladite molécule par ladite au moins une cellule ou bien ledit récipient R ne contenant pas un tel au moins un stimulant,
(c) lorsque le récipient ne contient pas au préalable au moins un stimulant de sécrétion, introduire dans ledit récipient R à l'aide dudit dispositif automatisé d'aspiration/refoulement, ledit au moins un stimulant de sécrétion contenu dans un récipient E, et
(d) laisser incuber la quantité définie d'échantillon et ledit au moins un stimulant, formant un mélange, pour la sécrétion de ladite molécule par ladite au moins une cellule (i) dans ledit récipient R ou (ii) dans un autre récipient S après aspiration et refoulement dudit mélange à l'aide dudit dispositif automatisé d'aspiration/refoulement, et
(e) détecter la réponse cellulaire immune, dans laquelle la détection de la réponse cellulaire immune indique la présence d'une réponse cellulaire immune spécifique dudit au moins un stimulant de sécrétion, **caractérisée** en ce ledit échantillon biologique est un échantillon de sang total qui a été préalablement prélevé chez un organisme animal.

2. Procédé de détection d'une réponse cellulaire immune selon la revendication 1, **caractérisé en ce que** le stimulant de sécrétion est un stimulant non spécifique ou un stimulant spécifique d'un microorganisme pathogène.

3. Procédé de détection d'une réponse cellulaire immune selon la revendication 1 ou 2, **caractérisé en ce que** ledit au moins un stimulant de sécrétion est un antigène hautement spécifique pour *Mycobacterium tuberculosis,* lequel peut être absent du vaccin BCG (Bacille Calmette-Guérin).

4. Procédé de détection d'une réponse cellulaire immune selon la revendication 3, **caractérisé en ce que** ledit au moins un stimulant de sécrétion est choisi parmi les antigènes ESAT-6 (6-kDa early secretory antigenic target), CFP-10 (10-kDa culture filtrate protein) et TB7.7 (Tuberculosis 7.7).

5. Procédé de détection d'une réponse cellulaire immune selon l'une quelconque des revendications 1 à 4, **caractérisé en ce que** ladite au moins une cellule est une cellule susceptible de secréter au moins une cytokine.

6. Procédé de détection d'une réponse cellulaire immune selon la revendication 5, **caractérisé en ce que** ladite au moins une cytokine est choisie parmi les interleukines, les chimiokines, le facteur de nécrose tumorale α (TNFα) et l'interféron gamma (IFNy), de préférence l'interféron gamma.

7. Procédé de détection d'une réponse cellulaire immune selon la revendication 1 ou 2, **caractérisé en ce que** les cellules sont des cellules B susceptibles de secréter au moins un anticorps.

8. Procédé de détection d'une réponse cellulaire immune selon l'une quelconque des revendications 1 à 7, **caractérisé en ce que** l'échantillon biologique est un échantillon d'origine humaine, bovine, canine, féline ou équine.

9. Procédé de détection d'une réponse cellulaire immune selon l'une quelconque des revendication 1 à 8, **caractérisé en ce que** le récipient R est non stérile après introduction de la quantité définie d'échantillon.

10. Procédé de détection d'une réponse cellulaire immune selon l'une quelconque des revendications précédentes, **caractérisé en ce que** la durée de l'étape d'incubation est comprise entre 2 et 72h.

11. Procédé de détection d'une réponse cellulaire immune selon l'une quelconque des revendications 1 à 10, **caractérisé en ce que** l'étape (e) met en œuvre un partenaire de liaison à la molécule effectrice immune qui est un partenaire d'immunoessai et **en ce que** la révélation de la liaison ou non de ladite molécule est mise en œuvre par un test sandwich utilisant un autre partenaire de liaison à la molécule, de nature différente ou non, l'un des deux partenaires étant marqué.

12. Procédé de détection d'une réponse cellulaire immune selon l'une quelconque des revendications 1 à 10, **caractérisé en ce que** l'étape (e) met en œuvre un partenaire de liaison à la molécule effectrice immune qui est un partenaire d'immunoessai et **en ce que** la révélation de la liaison ou non de ladite molécule est mise en œuvre par un test en compétition utilisant un composé marqué entrant en compétition avec ladite molécule.

13. Procédé de détection d'une réponse cellulaire immune selon l'une quelconque des revendications 1 à 12, **caractérisé en ce que** les étapes (a) à (e) sont mises en œuvre avec le même instrument.

14. Procédé de détection d'une réponse cellulaire immune selon l'une quelconque des revendications 1 à 13, **caractérisé en ce que** les étapes (a) à (e) sont mises en œuvre successivement dans la même journée.

15. Procédé de détection d'une réponse cellulaire immune selon l'une quelconque des revendications 1 à 13, **caractérisé en ce que** les étapes (a) à (d) sont mises en œuvre un premier jour ou pendant plusieurs jours, et l'étape (e) est mise en œuvre ensuite unautre jour.

16. Procédé automatisé de stimulation d'au moins une cellule susceptible de sécréter une molécule effectrice immune pour la sécrétion de ladite molécule, ladite au moins une cellule étant contenue dans un échantillon biologique de sang total qui a été préalablement prélevé
chez un organisme animal, **caractérisé en ce qu'**il comprend ou consiste en les étapes consistant à :
(a) aspirer une quantité définie (calibrée) de l'échantillon biologique qui a été préalablement prélevé, par un dispositif automatisé d'aspiration/refoulement,
(b) refouler cette quantité définie, à l'aide dudit dispositif automatisé d'aspiration/refoulement, dans un récipient R, ledit récipient R contenant au préalable au moins un stimulant de sécrétion de ladite molécule par ladite au moins une cellule ou bien ledit récipient R ne contenant pas un tel au moins un stimulant,
(c) lorsque le récipient ne contient pas au préalable au moins un stimulant de sécrétion, introduire dans ledit récipient R à l'aide dudit dispositif automatisé d'aspiration/refoulement, ledit au moins un stimulant de sécrétion contenu dans un récipient E, et
(d) laisser incuber la quantité définie d'échantillon et ledit au moins un stimulant, formant un mélange, pour la sécrétion de ladite molécule par ladite au moins une cellule (i) dans ledit récipient R ou (ii) dans un autre récipient S après aspiration et refoulement dudit mélange à l'aide dudit dispositif automatisé d'aspiration/refoulement.

17. Procédé automatisé de stimulation selon la revendication 16, **caractérisé en ce que** le récipient R est non stérile après introduction de la quantité définie d'échantillon.

18. Procédé automatisé de stimulation selon la revendication 16 ou 17, **caractérisé en ce que** le stimulant de sécrétion est un stimulant non spécifique ou un stimulant spécifique d'un microorganisme pathogène

19. Procédé automatisé de stimulation selon l'une quelconque des revendications 16 à 18, **caractérisé en ce que** l'échantillon biologique est un échantillon d'origine humaine, bovine, canine, féline ou équine.

20. Utilisation d'un procédé de détection d'une réponse cellulaire immune selon l'une quelconque des revendications 1 à 15 ou du procédé automatique de stimulation selon l'une quelconque des revendications 16 à 19 pour le diagnostic de pathologies, pour les études de toxicité de médicaments et vaccins et pour déterminer le statut immunitaire d'un patient.

## Patentansprüche

1. Verfahren zum Nachweis einer Immunzellantwort in einer biologischen Probe tierischen Ursprungs, die mindestens eine Zelle enthalten kann, die mindestens ein Immuneffektormolekül sezerniert, das die aus Folgendem bestehenden Schritte umfasst:
(a) das Ansaugen einer definierten Menge der zuvor entnommenen biologischen Probe mittels einer automatisierten Ansaug-/Ablassvorrichtung,
(b) das Ablassen dieser definierten Menge mittels der automatisierten Ansaug-/Ablassvorrichtung in einen Behälter R, wobei der Behälter R vorher mindestens ein Stimulans der Sekretion des Moleküls durch die mindestens eine Zelle enthält oder auch der Behälter R ein solches mindestens ein Stimulans nicht enthält,
(c) wenn der Behälter zuvor nicht mindestens ein Sekretionsstimulans enthält, mittels der automatisierten Ansaug-/Ablassvorrichtung das Einführen des mindestens einen in einem Behälter E enthaltenen Sekretionsstimulans in den Behälter R und
(d) das Inkubierenlassen der definierten Probenmenge und des mindestens einen Stimulans unter Bildung einer Mischung zur Sekretion des Moleküls durch die mindestens eine Zelle (i) im Behälter R oder (ii) in einem anderen Behälter S nach einem Ansaugen und Ablassen der Mischung mittels der automatisierten Ansaug-/Ablassvorrichtung und
(e) das Nachweisen der Immunzellantwort, wobei der Nachweis der Immunzellantwort auf das Vorhandensein einer für das mindestens eine Sekretionsstimulans spezifischen Immunzellantwort hindeutet,
**dadurch gekennzeichnet, dass** es sich bei der biologischen Probe um eine Vollblutprobe handelt, die zuvor einem tierischen Organismus entnommen wurde.

2. Verfahren zum Nachweis einer Immunzellantwort nach Anspruch 1, **dadurch gekennzeichnet, dass** es sich beim Sekretionsstimulans um ein unspezifisches Stimulans oder ein für einen pathogenen Mikroorganismus spezifisches Stimulans handelt.

3. Verfahren zum Nachweis einer Immunzellantwort nach Anspruch 1 oder 2, **dadurch gekennzeichnet, dass** es sich bei dem mindestens einen Sekretionsstimulans um ein für *Mycobacterium tuberculosis* hochspezifisches Antigen handelt, das im BCG- (Bacille Calmette-Guerin-)Impfstoff fehlen kann.

4. Verfahren zum Nachweis einer Immunzellantwort nach Anspruch 3, **dadurch gekennzeichnet, dass** das mindestens eine Sekretionsstimulans aus den Antigenen ESAT-6 (6-kDa early secretory antigenic target), CFP-10 (10-kDa culture filtrate protein) und TB7.7 (Tuberculosis 7.7) ausgewählt ist.

5. Verfahren zum Nachweis einer Immunzellantwort nach einem der Ansprüche 1 bis 4, **dadurch gekennzeichnet, dass** es sich bei der mindestens einen Zelle um eine Zelle handelt, die mindestens ein Zytokin sezernieren kann.

6. Verfahren zum Nachweis einer Immunzellantwort nach Anspruch 5, **dadurch gekennzeichnet, dass** das mindestens eine Zytokin aus Interleukinen, Chemokinen, Tumor-Nekrose-Faktor α (TNFα) und Interferon gamma (IFNγ), vorzugsweise Interferon gamma, ausgewählt ist.

7. Verfahren zum Nachweis einer Immunzellantwort nach Anspruch 1 oder 2, **dadurch gekennzeichnet, dass** es sich bei den Zellen um B-Zellen handelt, die mindestens einen Antikörper sezernieren können.

8. Verfahren zum Nachweis einer Immunzellantwort nach einem der Ansprüche 1 bis 7, **dadurch gekennzeichnet, dass** es sich bei der biologischen Probe um eine Probe menschlichen, bovinen, caninen, felinen oder equinen Ursprungs handelt.

9. Verfahren zum Nachweis einer Immunzellantwort nach einem der Ansprüche 1 bis 8, **dadurch gekennzeichnet, dass** der Behälter R nach dem Einführen der definierten Probenmenge unsteril ist.

10. Verfahren zum Nachweis einer Immunzellantwort nach einem der vorhergehenden Ansprüche, **dadurch gekennzeichnet, dass** die Dauer des Inkubationsschritts zwischen 2 und 72 h liegt.

11. Verfahren zum Nachweis einer Immunzellantwort nach einem der Ansprüche 1 bis 10, **dadurch gekennzeichnet, dass** bei Schritt (e) ein Partner für die Bindung am Immuneffektormolekül verwendet wird, bei dem es sich um einen Immunoassay-Partner handelt, und dadurch, dass die Offenbarung der Bindung oder nicht des Moleküls durch einen Sandwich-Assay erfolgt, bei dem ein anderer Partner unterschiedlicher Art oder nicht für die Bindung am Molekül verwendet wird, wobei einer der zwei Partner markiert ist.

12. Verfahren zum Nachweis einer Immunzellantwort nach einem der Ansprüche 1 bis 10, **dadurch gekennzeichnet, dass** bei Schritt (e) ein Partner für die Bindung am Immuneffektormolekül verwendet wird, bei dem es sich um einen Immunoassay-Partner handelt, und dadurch, dass die Offenbarung der Bindung oder nicht des Moleküls durch einen kompetitiven Assay erfolgt, bei dem eine markierte Verbindung verwendet wird, die mit dem Molekül konkurriert.

13. Verfahren zum Nachweis einer Immunzellantwort nach einem der Ansprüche 1 bis 12, **dadurch gekennzeichnet, dass** die Schritte (a) bis (e) mit demselben Gerät erfolgen.

14. Verfahren zum Nachweis einer Immunzellantwort nach einem der Ansprüche 1 bis 13, **dadurch gekennzeichnet, dass** die Schritte (a) bis (e) nacheinander am selben Tag erfolgen.

15. Verfahren zum Nachweis einer Immunzellantwort nach einem der Ansprüche 1 bis 13, **dadurch gekennzeichnet, dass** die Schritte (a) bis (d) an einem ersten Tag oder in Verlauf mehrerer Tage erfolgen und Schritt (d) anschließend an einem anderen Tag erfolgt.

16. Automatisiertes Verfahren zur Stimulation mindestens einer Zelle, die ein Immuneffektormolekül sezernieren kann, zur Sekretion des Moleküls, wobei die mindestens eine Zelle in einer biologischen Vollblutprobe enthalten ist, die zuvor einem tierischen Organismus entnommen wurde, **dadurch gekennzeichnet, dass** es die aus Folgendem bestehenden Schritte umfasst oder daraus besteht:
(a) das Ansaugen einer (kalibrierten) definierten Menge der zuvor entnommenen biologischen Probe mittels einer automatisierten Ansaug-/Ablassvorrichtung,
(b) das Ablassen dieser definierten Menge mittels der automatisierten Ansaug-/Ablassvorrichtung in einen Behälter R, wobei der Behälter R vorher mindestens ein Stimulans der Sekretion des Moleküls durch die mindestens eine Zelle enthält oder auch der Behälter R ein solches mindestens ein Stimulans nicht enthält,
(c) wenn der Behälter zuvor nicht mindestens ein Sekretionsstimulans enthält, mittels der automatisierten Ansaug-/Ablassvorrichtung das Einführen des mindestens einen in einem Behälter E enthaltenen Sekretionsstimulans in den Behälter R und
(d) das Inkubierenlassen der definierten Probenmenge und des mindestens einen Stimulans unter Bildung einer Mischung zur Sekretion des Moleküls durch die mindestens eine Zelle (i) im Behälter R oder (ii) in einem anderen Behälter S nach einem Ansaugen und Ablassen der Mischung mittels der automatisierten Ansaug-/Ablassvorrichtung.

17. Automatisiertes Stimulationsverfahren nach Anspruch 16, **dadurch gekennzeichnet, dass** der Behälter R nach dem Einführen der definierten Probenmenge unsteril ist.

18. Automatisiertes Stimulationsverfahren nach Anspruch 16 oder 17, **dadurch gekennzeichnet, dass** es sich beim Sekretionsstimulans um ein unspezifisches Stimulans oder ein für einen pathogenen Mikroorganismus spezifisches Stimulans handelt.

19. Automatisiertes Stimulationsverfahren einem der Ansprüche 16 bis 18, **dadurch gekennzeichnet, dass** es sich bei der biologischen Probe um eine Probe menschlichen, bovinen, caninen, felinen oder equinen Ursprungs handelt.

20. Verwendung eines Verfahrens zum Nachweis einer Immunzellantwort nach einem der Ansprüche 1 bis 15 oder des automatischen Stimulationsverfahrens nach einem der Ansprüche 16 bis 19 zur Diagnose von Krankheiten, für Untersuchungen der Toxizität von Medikamenten und Impfstoffen und zur Bestimmung des Immunstatus eines Patienten.

## Claims

1. A method for detecting an immune cellular response in a biological sample of animal origin, which may contain at least one cell which secretes at least one immune effector molecule, comprising the steps consisting in:
(a) suctioning a defined amount of the biological sample as previously taken by means of an automated suction/discharge device,
(b) discharging this defined amount, using said automated suction/discharge device, into a container R, said container R containing, beforehand, at least one stimulant for secretion of said molecule by said at least one cell, or else said container R not containing such an at least one stimulant,
(c) when the container does not contain, beforehand, at least one secretion stimulant, introducing into said container R said at least one secretion stimulant contained in a container E, using said automated suction/discharge device, and
(d) allowing the defined amount of sample and said at least one stimulant to incubate, forming a mixture, for the secretion of said molecule by said at least one cell (i) in said container R or (ii) in another container S after suctioning and discharge of said mixture using said automated suction/discharge device, and
(e) detecting the immune cellular response, wherein the detection of the immune cellular response indicates the presence of an immune cellular response specific for said at least one secretion stimulant,
**characterized in that** said biological sample is a total blood sample previously taken from an animal organism.

2. The method for detecting an immune cellular response as claimed in claim 1, **characterized in that** the secretion stimulant is a nonspecific stimulant or a stimulant specific for a pathogenic microorganism.

3. The method for detecting an immune cellular response as claimed in claim 1 or 2, **characterized in that** said at least one secretion stimulant is an antigen highly specific for *Mycobacterium tuberculosis,* which may be absent from the BCG (Bacillus Calmette-Guerin) vaccine.

4. The method for detecting an immune cellular response as claimed in claim 3, **characterized in that** said at least one secretion stimulant is chosen from the ESAT-6 (6-kDa early secretory antigenic target), CFP-10 (10-kDa culture filtrate protein) and TB7.7 (Tuberculosis 7.7) antigens.

5. The method for detecting an immune cellular response as claimed in any one of claims 1 to 4, **characterized in that** said at least one cell is a cell capable of secreting at least one cytokine.

6. The method for detecting an immune cellular response as claimed in claim 5, **characterized in that** said at least one cytokine is chosen from interleukins, chemokines, tumor necrosis factor α (TNFα) and interferon gamma (IFNy), preferably interferon gamma.

7. The method for detecting an immune cellular response as claimed in claim 1 or 2, **characterized in that** the cells are B cells capable of secreting at least one antibody.

8. The method for detecting an immune cellular response as claimed in any one of claims 1 to 7, **characterized in that** the biological sample is a biological sample of human, bovine, canine, feline or equine origin.

9. The method for detecting an immune cellular response as claimed in any one of claims 1 to 8, **characterized in that** the container R is nonsterile after introduction of the defined amount of sample.

10. The method for detecting an immune cellular response as claimed in any one of the preceding claims, **characterized in that** the duration of the incubation step is between 2 and 72 h.

11. The method for detecting an immune cellular response as claimed in any one of claims 1 to 10, **characterized in that** step (e) uses a partner for binding to the immune effector molecule which is an immunoassay partner and **in that** the revealing of the binding or nonbinding of said molecule is carried out by means of a sandwich assay using another partner for binding to the molecule, which may or may not be different in nature, one of the two partners being labeled.

12. The method for detecting an immune cellular response as claimed in any one of claims 1 to 10, **characterized in that** step (e) uses a partner for binding to the immune effector molecule which is an immunoassay partner and **in that** the revealing of the binding or nonbinding of said molecule is carried out by means of a competition assay using a labeled compound which competes with said molecule.

13. The method for detecting an immune cellular response as claimed in any one of claims 1 to 12, **characterized in that** steps (a) to (e) are carried out with the same instrument.

14. The method for detecting an immune cellular response as claimed in any one of claims 1 to 13, **characterized in that** steps (a) to (e) are carried out successively on the same day.

15. The method for detecting an immune cellular response as claimed in any one of claims 1 to 13, **characterized in that** steps (a) to (d) are carried out on a first day or over the course of several days, and step (e) is then carried out on another day.

16. An automated method for stimulating at least one cell capable of secreting an immune effector molecule for the secretion of said molecule, said at least one cell being present in a total blood sample previously taken from an animal organism, **characterized in that** it comprises or consists of the steps consisting in:
(a) suctioning a defined (calibrated) amount of the biological sample as previously taken by means of an automated suction/discharge device,
(b) discharging this defined amount, using said automated suction/discharge device, into a container R, said container R containing, beforehand, at least one stimulant for secretion of said molecule by said at least one cell, or else said container R not containing such an at least one stimulant,
(c) when the container does not contain, beforehand, at least one secretion stimulant, introducing into said container R said at least one secretion stimulant contained in a container E, using said automated suction/discharge device, and
(d) allowing the defined amount of sample and said at least one stimulant to incubate, forming a mixture, for the secretion of said molecule by said at least one cell (i) in said container R or (ii) in another container S after suctioning and discharge of said mixture using said automated suction/discharge device.

17. The automated stimulation method as claimed in claim 16, **characterized in that** the container R is nonsterile after introduction of the defined amount of sample.

18. The automated stimulation method as claimed in any one of claim 16 or 17, **characterized in that** the secretion stimulant is a nonspecific stimulant or a stimulant specific for a pathogenic microorganism.

19. The automated stimulation method as claimed in any one of claims 16 to 18, **characterized in that** the biological sample is a sample of human, bovine, canine, feline or equine origin.

20. The use of a method for detecting an immune cellular response as claimed in any one of claims 1 to 15 or of the automatic stimulation method as claimed in any one of claims 17 to 19, for the diagnosis of pathological conditions, for medicament and vaccine toxicity studies and for determining the immune status of a patient.
